# EUROPEAN PATENT APPLICATION

(11) **EP 4 825 363 A1**
(43) Date of publication of application: **21.10.2026**
(21) Application number: 24903626.0
(22) Date of filing: 06.12.2024
(51) Int. Cl.: A61K 8/19, A61K 8/02, A61K 8/36, A61K 8/44, A61K 8/365, A61Q 5/02, A61Q 19/10

(54) **POWDER OR GRANULAR CARBONIC ACID FOAMING COMPOSITION AND POWDER OR GRANULAR DETERGENT COMPOSITION**

(30) Priority: 14.12.2023 JP 2023211334; 14.12.2023 JP 2023211332
(71) Applicant: Kao Corporation, Tokyo 103-8210 (JP)
(72) Inventor: IKEDA Manato, Tokyo 103-8210 (JP); HAYASHIDA Yumi, Tokyo 103-8210 (JP); TAKEDA Saki, Tokyo 103-8210 (JP)
(74) Representative: Hoffmann Eitle
(86) International application number: PCT/JP2024/043206
(87) International publication number: WO 2025/126971

(57) **Abstract**

Provided is a powder or granular carbonic acid foaming composition containing a carbonate and an organic acid, wherein particles having a particle size of 100 µm or larger are contained at 30% by mass or more, and a content of particles having a particle size of 100 µm or larger and having a hardness of 5 gf or higher is 0.10% by number or less.

## Description

### Field of the Invention

The present invention relates to a powder or granular carbonic acid foaming composition and a powder or granular cleansing composition.

### Background of the Invention

Foam-type shampoos, face washes, and body soaps have been receiving attention in recent years from the viewpoint of being easily available.

In these circumstances, a foamable cosmetic composition which generates fine foam of carbonic acid gas has been reported by exploiting the property that the addition of a small amount of water to a mixture of a carbonate and an organic acid generates carbonic acid gas (JP-A-1989-290615: Patent Literature 1). The patent literature discloses that this foamable cosmetic composition can be used as a facial soap, a shaving foam, a shampoo, or a body soap. However, the foamable cosmetic composition described in Patent Literature 1 generates carbonic acid gas during preservation if water is present, even in a very small amount, in the mixture of the carbonate and the organic acid. Furthermore, since water is generated as a by-product of the reaction, a chain reaction occurs, disadvantageously resulting in the swelling of a packaging material or reduced foamability upon application.

To solve this problem, a technique for improving preservation stability has been reported. For example, JP-A-2009-62319 (Patent Literature 2) discloses foamable granules containing an oil component and having a particle size of from 150 µm to 1 500 µm, whereby preservation stability is improved.

JP-A-2009-155213 (Patent Literature 3) discloses a bath agent composition containing the following components (A) to (C): (A) 25 to 55% by mass of a carbonic acid alkali metal salt containing 50% or more of particles having a particle size of 180 µm or larger, (B) 40 to 70% by mass of an organic acid containing 50% or more of particles having a particle size of 180 µm or larger, and (C) 0.01 to 10% by mass of a poorly water-soluble metal oxide, whereby preservation stability is favorable.

### Summary of the Invention

The present invention relates to the following [1] to [4].
[1] A powder or granular carbonic acid foaming composition containing
   a carbonate and an organic acid, wherein
   particles having a particle size of 100 µm or larger are contained at 30% by mass or more, and
   a content of particles having a particle size of 100 µm or larger and having a hardness of 5 gf or higher is 0.10% by number or less.
[2] A method for producing the powder or granular carbonic acid foaming composition according to [1], containing
   a step of granulating at least one of the carbonate and the organic acid.
[3] A powder or granular cleansing composition containing
   a carbonate, an organic acid, and a surfactant, wherein
   particles having a particle size of 100 µm or larger are contained at 30% by mass or more, and
   a content of particles having a particle size of 100 µm or larger and having a hardness of 5 gf or higher is 0.10% by number or less.
[4] A method for producing the powder or granular cleansing composition according to [3], containing
   a step of granulating at least one of the carbonate and the organic acid.

### Detailed Description of the Invention

However, the technique described in JP-A-2009-62319 (Patent Literature 2) employs a compression granulation method and therefore reduces the instant solubility of granules. Hence, in the case of applying this technique to a carbonic acid foaming composition to be used in making lather with a shampoo or the like by hand, a component left undissolved has been found to cause roughness, which deteriorates texture upon application and impairs feeling in use. The technique of JP-A-2009-155213 (Patent Literature 3) relates to a bath agent. When a bath agent composition is dissolved on the surface of hot bath water, carbonic acid gas dissipates without being sufficiently dissolved in the hot bath water. Therefore, in order to dissolve the bath agent composition in hot bath water, the bath agent composition needs to be immersed in the hot bath water so as to stay in the hot bath water for a given time. Accordingly, this technique is predicated on use of starting material particles having a large particle size to a given degree as particles constituting the bath agent composition. By contrast, a carbonic acid foaming composition to be used in making lather by hand requires instant solubility which permits instant dissolution in a short time in a small amount of water. Therefore, particles having a large particle size, if used in the carbonic acid foaming composition, have poor solubility, causing a roughness problem, as in the case described above. Unlike a bath agent, the carbonic acid foaming composition is required to offer delicate feel (texture) because the composition is applied to hair or the body and has direct skin contact.

Thus, an object of the present invention is to provide a powder or granular carbonic acid foaming composition and a powder or granular cleansing composition which have high preservation stability, exhibit reduced roughness in texture, and offer favorable feeling in use.

As a result of conducting studies, the present inventors found that the object can be attained by a powder or granular carbonic acid foaming composition which is a carbonic acid foaming composition containing a carbonate and an organic acid, wherein particles having a particle size of a predetermined value or larger are contained in a predetermined amount or more, and a content of particles having a particle size and a hardness of predetermined values or more is a predetermined amount or less.

As a result of conducting studies, the present inventors also found that the object can be attained by a powder or granular cleansing composition which is a cleansing composition containing a carbonate, an organic acid, and a surfactant, wherein particles having a particle size of a predetermined value or larger are contained in a predetermined amount or more, and a content of particles having a particle size and a hardness of predetermined values or more is a predetermined amount or less.

The first embodiment of the present invention relates to a powder or granular carbonic acid foaming composition containing a carbonate and an organic acid, wherein particles having a particle size of 100 µm or larger are contained at 30% by mass or more, and a content of particles having a particle size of 100 µm or larger and having a hardness of 5 gf or higher is 0.10% by number or less.

The second embodiment of the present invention relates to a powder or granular cleansing composition containing a carbonate, an organic acid, and a surfactant, wherein particles having a particle size of 100 µm or larger are contained at 30% by mass or more, and a content of particles having a particle size of 100 µm or larger and having a hardness of 5 gf or higher is 0.10% by number or less.

The present invention can provide a powder or granular carbonic acid foaming composition and a powder or granular cleansing composition which have high preservation stability, exhibit reduced roughness in texture, and offer favorable feeling in use.

### [First embodiment: powder or granular carbonic acid foaming composition]

The powder or granular carbonic acid foaming composition according to the first embodiment of the present invention contains
a carbonate and an organic acid, wherein
particles having a particle size of 100 µm or larger are contained at 30% by mass or more, and
a content of particles having a particle size of 100 µm or larger and having a hardness of 5 gf or higher is 0.10% by number or less.

The powder or granular carbonic acid foaming composition (hereinafter, also simply referred to as the carbonic acid foaming composition) according to the first embodiment of the present invention is used for, for example, a surfactant-containing composition such as a shampoo or a conditioning agent to form the surfactant-containing composition into a dosage form of foam. Thus, the powder or granular carbonic acid foaming composition according to the first embodiment of the present invention is applied in combination with a surfactant-containing composition to the surface of the human body including the skin and hair. Hence, the powder or granular carbonic acid foaming composition is required to offer favorable feeling in use without roughness in texture in making lather, and needs to be instantly dissolved in a short time. Thus, particles constituting the carbonic acid foaming composition desirably have a small particle size. However, if the carbonic acid foaming composition has a small particle size, a moisture absorbent added thereto has been found to be insufficiently effective, be unable to suppress the generation of carbonic acid gas in a packaging material, and render preservation stability poor. Accordingly, in order to suppress the generation of carbonic acid gas during preservation, the carbonate or the organic acid needs to have a large particle size. As a result of conducting extensive studies to solve these conflicting demands, the present inventors completed the present invention by finding that the hardness and the particle size of particles constituting the carbonic acid foaming composition are deeply involved in texture upon application and preservation stability, and finding that both the texture and the preservation stability can be achieved by adjusting the hardness or the particle size of the particles in the carbonic acid foaming composition.

The powder or granular carbonic acid foaming composition according to the first embodiment of the present invention can reduce roughness in texture upon application and produce favorable feeling in use, by containing a predetermined amount or more of particles having a particle size of 100 µm or larger, and setting the content of particles having a particle size of 100 µm or larger and having a hardness of 5 gf or higher to a predetermined percent by number or less.

The powder or granular carbonic acid foaming composition according to the first embodiment of the present invention may contain a surfactant so long as the effect of the surfactant-containing composition to be used in combination therewith is not inhibited. The content of the surfactant may be less than 10% by mass, may be 7% by mass or less, or may be 5% by mass or less, and may be 1% by mass or more, may be 2% by mass or more, or may be 3% by mass or more.

The surfactant-containing composition will be described in detail in the section about a method for using the powder or granular carbonic acid composition.

The surfactant to be used in the powder or granular carbonic acid foaming composition according to the first embodiment of the present invention can be the same as one to be used in the powder or granular cleansing composition according to the second embodiment of the present invention mentioned later.

### <Carbonate>

The powder or granular carbonic acid foaming composition according to the first embodiment of the present invention contains a carbonate.

Examples of the carbonate used in the present invention include: carbonic acid dialkali metal salts such as sodium carbonate and potassium carbonate; and carbonic acid hydrogen alkali metal salts such as sodium bicarbonate and potassium bicarbonate. One or more of these carbonates can be used.

Among those described above, the carbonate preferably contains one or more members selected from the group consisting of sodium carbonate (Na₂CO₃) and sodium bicarbonate (NaHCO₃), and more preferably contains sodium bicarbonate, from the viewpoint of improvement in foamability. The content of the one or more members selected from the group consisting of sodium carbonate and sodium bicarbonate in the carbonate is preferably 80% by mass or more, more preferably 90% by mass or more, and preferably 100% by mass or less, more preferably 100% by mass, from the viewpoint of improvement in foamability.

The content of the carbonate in the powder or granular carbonic acid foaming composition is preferably 15% by mass or more, more preferably 25% by mass or more, further more preferably 30% by mass or more, even more preferably 35% by mass or more, from the viewpoint of improvement in foamability. The content is preferably 65% by mass or less, more preferably 60% by mass or less, further more preferably 55% by mass or less, from the viewpoint of improvement in long-lasting foam. The content of the carbonate in the powder or granular carbonic acid foaming composition is preferably 15% by mass or more and 65% by mass or less, more preferably 25% by mass or more and 60% by mass or less, further more preferably 30% by mass or more and 55% by mass or less, even more preferably 35% by mass or more and 55% by mass or less.

The carbonate used in the present invention may be contained as particles of a starting material itself (hereinafter, also referred to as "starting material particles") in the powder or granular carbonic acid foaming composition according to the first embodiment of the present invention, or may be contained as particles granulated (hereinafter, also referred to as "granulated particles"), if necessary, with an additional component, or may be contained as a mixture thereof. When the carbonate is granulated particles, the carbonate may be granulated alone or may be granulated together with a component other than the carbonate. The granulated particles preferably contain no organic acid from the viewpoint of preservation stability. In this context, the phrase "contain no" means being substantially free thereof, and the amount of the organic acid in the granulated particles is preferably less than 1% by mass, more preferably 0% by mass. The carbonate is preferably granulated together with an additional component other than the organic acid, for example, one or more members selected from the group consisting of an excipient, a water-soluble polymer (a binder and a feel modifier), and a moisture absorbent, and particularly preferably granulated with all components other than the organic acid. The granulation together with a component other than the organic acid can reduce the amount of a fine powder derived from the component other than the organic acid, and can prevent choking caused by scattered fine powder upon application of the carbonic acid foaming composition.

### <Organic acid>

The powder or granular carbonic acid foaming composition according to the first embodiment of the present invention contains an organic acid.

Examples of the organic acid used in the present invention include citric acid, tartaric acid, malic acid, malonic acid, pyridonecarboxylic acid, succinic acid, fumaric acid, adipic acid, glutaric acid, and ascorbic acid. One or more of these organic acids can be used. Among those described above, the organic acid preferably contains one or more members selected from the group consisting of citric acid, succinic acid, tartaric acid, and ascorbic acid, and more preferably contains citric acid, from the viewpoint of foamability, and solubility in water. The content of the one or more members selected from the group consisting of citric acid, succinic acid, tartaric acid, and ascorbic acid in the organic acid is preferably 80% by mass or more, more preferably 90% by mass or more, and preferably 100% by mass or less, more preferably 100% by mass, from the viewpoint of improvement in foamability.

The content of the organic acid in the powder or granular carbonic acid foaming composition is preferably 5% by mass or more, more preferably 10% by mass or more, further more preferably 15% by mass or more, even more preferably 20% by mass or more, from the viewpoint of improvement in foamability. The content is preferably 60% by mass or less, more preferably 50% by mass or less, further more preferably 45% by mass or less, even more preferably 40% by mass or less, even more preferably 35% by mass or less, from the viewpoint of improvement in long-lasting foam. The content of the organic acid in the powder or granular carbonic acid foaming composition is preferably 5% by mass or more and 60% by mass or less, more preferably 10% by mass or more and 50% by mass or less, further more preferably 10% by mass or more and 45% by mass or less, even more preferably 15% by mass or more and 40% by mass or less, even more preferably 20% by mass or more and 35% by mass or less.

The organic acid used in the present invention may be contained as particles of a starting material itself (starting material particles) in the powder or granular carbonic acid foaming composition according to the first embodiment of the present invention, or may be contained as particles granulated (granulated particles), if necessary, with an additional component, or may be contained as a mixture thereof. When the organic acid is granulated particles, the organic acid may be granulated alone or may be granulated together with a component other than the organic acid. The granulated particles preferably contain no carbonate from the viewpoint of preservation stability. In this context, the phrase "contain no" means being substantially free thereof, and the amount of the carbonate in the granulated particles is preferably less than 1% by mass, more preferably 0% by mass. The organic acid is preferably granulated together with a component other than the carbonate, for example, one or more members selected from the group consisting of an excipient, a binder, and a moisture absorbent, and particularly preferably granulated with all components other than the carbonate. The granulation together with a component other than the carbonate can reduce the amount of a fine powder derived from the component other than the carbonate, and can prevent choking caused by scattered fine powder upon application of the carbonic acid foaming composition.

In the powder or granular carbonic acid foaming composition, the mass ratio of the organic acid to the carbonate [organic acid/carbonate] is preferably 0.05 or more, more preferably 0.1 or more, further more preferably 0.2 or more, and preferably 10 or less, more preferably 5.0 or less, further more preferably 1.0 or less, from the viewpoint of foamability. The mass ratio of the organic acid to the carbonate [organic acid/carbonate] is preferably 0.05 or more and 10 or less, more preferably 0.1 or more and 5.0 or less, further more preferably 0.2 or more and 1.0 or less.

The total amount of the carbonate and the organic acid in the powder or granular carbonic acid foaming composition is preferably 50% by mass or more, more preferably 60% by mass or more, further more preferably 65% by mass or more, from the viewpoint of foamability, and preferably 90% by mass or less, more preferably 85% by mass or less, further more preferably 80% by mass or less, from the viewpoint of improvement in long-lasting foam. The total amount of the carbonate and the organic acid in the powder or granular carbonic acid foaming composition is preferably 50% by mass or more and 90% by mass or less, more preferably 60% by mass or more and 85% by mass or less, further more preferably 65% by mass or more and 80% by mass or less.

### <Moisture absorbent>

The powder or granular carbonic acid foaming composition according to the first embodiment of the present invention preferably further contains a moisture absorbent.

Examples of the moisture absorbent used in the present invention include alkaline earth metal oxides such as magnesium oxide, calcium oxide, and zinc oxide. Among those described above, magnesium oxide is preferred from the viewpoint of improvement in preservation stability and foamability.

The content of the moisture absorbent in the powder or granular carbonic acid foaming composition is preferably 0.01% by mass or more, more preferably 0.1% by mass or more, further more preferably 1% by mass or more, even more preferably 2% by mass or more, even more preferably 4% by mass or more, from the viewpoint of improvement in preservation stability. The content is preferably 15% by mass or less, more preferably 12% by mass or less, further more preferably 10% by mass or less, from the viewpoint of improvement in foamability. The content of the moisture absorbent in the powder or granular carbonic acid foaming composition is preferably 0.01% by mass or more and 15% by mass or less, more preferably 0.1% by mass or more and 12% by mass or less, further more preferably 1% by mass or more and 12% by mass or less, even more preferably 2% by mass or more and 10% by mass or less, even more preferably 4% by mass or more and 10% by mass or less.

### <Other components>

The powder or granular carbonic acid foaming composition according to the first embodiment of the present invention may contain components which are generally used in powder or granular carbonic acid foaming compositions, so long as the object of the present invention is not impaired. Examples of such other components include excipients, water-soluble polymers, thickeners, natural dyes, moisturizers, antiinflammatory agents, germicides, antiperspirants, antioxidants, fragrances, and mixtures thereof.

The powder or granular carbonic acid foaming composition according to the first embodiment of the present invention is preferably substantially free of an oil component such as a fragrance from the viewpoint of foamability, and the content of the oil component is preferably less than 2% by mass, more preferably 1.5% by mass or less, further more preferably less than 1.0% by mass, even more preferably 0.5% by mass or less, even more preferably 0.1% by mass or less, even more preferably 0% by mass, in the powder or granular carbonic acid foaming composition.

Examples of the excipient used in the present invention include: inorganic powders such as silicic acid, silicic anhydride (silica), magnesium silicate, calcium silicate, aluminum silicate, barium silicate, strontium silicate, diatomaceous earth, talc, sericite, mica, kaolin, montmorillonite, clay, bentonite, vermiculite, titanium oxide-coated mica (mica titanium), bismuth oxychloride, boron nitride, zirconium oxide, titanium oxide, low-order titanium oxide, tungstic acid metal salts, hydroxyapatite, zeolite, ceramic powders, chlorohydroxyaluminum, aluminum chloride, aluminum sulfate, basic aluminum bromide, basic aluminum iodide, chlorohydroxyaluminum zirconium, zinc sulfate, basic aluminum zinc lactate, aluminum oxide, calcium sulfate, barium sulfate, magnesium sulfate, colcothar, black iron oxide, yellow iron oxide, ultramarine, iron blue, chromium oxide, chromium hydroxide, calamine, and carbon black; monosaccharides such as glucose, fructose, galactose, and mannose; disaccharides such as lactose, trehalose, and maltose; starches such as corn starch and potato starch; and sugar alcohols such as mannitol, maltitol, xylitol, and erythritol. One or more of these excipients can be used.

The content of the excipient in the powder or granular carbonic acid foaming composition is preferably 5% by mass or more, more preferably 10% by mass or more, and preferably 50% by mass or less, more preferably 40% by mass or less, further more preferably 30% by mass or less, from the viewpoint of solubility, granulability, foamability, and preservation stability. The content of the excipient in the powder or granular carbonic acid foaming composition is, preferably 5% by mass or more and 50% by mass or less, more preferably 10% by mass or more and 40% by mass or less, further more preferably 10% by mass or more and 30% by mass or less.

The water-soluble polymer used in the present invention is preferably derived from a polysaccharide from the viewpoint of improving feeling in use. Such a water-soluble polymer can be used as a feel modifier and a binder mentioned later.

Examples of the water-soluble polymer used in the present invention include: semisynthetic water-soluble polymers such as hydroxyethylcellulose, hydroxymethylcellulose, carboxymethylcellulose, cationized hydroxyethylcellulose, cationized carboxymethylcellulose, cationized xanthan gum, cationized carrageenan, and cationized guar gum; and natural water-soluble polymers such as xanthan gum, carrageenan, and guar gum. One or more of these water-soluble polymers can be used. Among those described above, the water-soluble polymer is preferably a water-soluble cationized polysaccharide, more preferably one or more members selected from the group consisting of cationized hydroxyethylcellulose, cationized carboxymethylcellulose, cationized xanthan gum, cationized carrageenan, and cationized guar gum, further more preferably one or more members selected from the group consisting of cationized hydroxyethylcellulose, cationized carboxymethylcellulose, and cationized guar gum, from the viewpoint of stabilizing foam generated upon contact with a surfactant-containing composition and from the viewpoint of improving feeling in use when added to a surfactant-containing composition such as a hair cosmetic for use.

The content of the water-soluble polymer in the powder or granular carbonic acid foaming composition is preferably 0.1% by mass or more, more preferably 1.0% by mass or more, further more preferably 2.5% by mass or more, and preferably 30% by mass or less, more preferably 20% by mass or less, further more preferably 10% by mass or less, even more preferably 5.0% by mass or less, from the viewpoint of stabilizing foam generated by addition to a surfactant-containing composition and from the viewpoint of improving feeling in use for use by addition to a surfactant-containing composition such as a hair cosmetic. The content of the water-soluble polymer in the powder or granular carbonic acid foaming composition is preferably 0.1% by mass or more and 30% by mass or less, more preferably 1.0% by mass or more and 20% by mass or less, further more preferably 1.0% by mass or more and 10% by mass or less, even more preferably 2.5% by mass or more and 5.0% by mass or less.

The content of the feel modifier in the powder or granular carbonic acid foaming composition is preferably 0.01% by mass or more, more preferably 0.3% by mass or more, further more preferably 0.8% by mass or more, even more preferably 1.3% by mass or more, even more preferably 2.3% by mass or more, and preferably 29.8% by mass or less, more preferably 25.3% by mass or less, further more preferably 19.8% by mass or less, even more preferably 9.8% by mass or less, even more preferably 4.8% by mass or less, from the viewpoint of stabilizing foam generated upon contact with a surfactant-containing composition and from the viewpoint of improving feeling in use for use by addition to a surfactant-containing composition such as a hair cosmetic. The content of the feel modifier in the powder or granular carbonic acid foaming composition is preferably 0.01% by mass or more and 29.8% by mass or less, more preferably 0.3% by mass or more and 25.3% by mass or less, further more preferably 0.8% by mass or more and 19.8% by mass or less, even more preferably 1.3% by mass or more and 9.8% by mass or less, even more preferably 2.3% by mass or more and 4.8% by mass or less.

### <Powder or granular carbonic acid foaming composition>

In the powder or granular carbonic acid foaming composition according to the first embodiment of the present invention, particles having a particle size of 100 µm or larger are contained at 30% by mass or more, and the content of particles having a particle size of 100 µm or larger and having a hardness of 5 gf or higher is 0.10% by number or less.

Specifically, in the powder or granular carbonic acid foaming composition according to the first embodiment of the present invention, the particles having a particle size of 100 µm or larger are contained at 30% by mass or more in particles constituting the powder or granular carbonic acid foaming composition, and the content of particles having a particle size of 100 µm or larger and having a hardness of 5 gf or higher is 0.10% by number or less in particles constituting the powder or granular carbonic acid foaming composition.

The particles having a particle size of 100 µm or larger are contained at 30% by mass or more, whereby preservation stability can be enhanced. The content of particles having a particle size of 100 µm or larger and having a hardness of 5 gf or higher is set to 0.10% by number or less, whereby roughness in texture is reduced, and feeling in use can be rendered favorable.

At least a part of the particles having a particle size of 100 µm or larger is preferably granulated particles. The content of the granulated particles in the particles having a particle size of 100 µm or larger is preferably more than 55% by number, more preferably more than 60% by number, further more preferably more than 65% by number, even more preferably more than 70% by number, and even more preferably, all of the particles are the granulated particles, from the viewpoint of improving feeling in use and, particularly, reducing roughness in texture, though the particles of a starting material itself may be partially contained from the viewpoint of production efficiency. Since the hardness of the granulated particles can be easily adjusted, the content of the 100 µm or larger particles having a hardness of 5 gf or higher can be adjusted.

The content of the granulated particles among the particles having a particle size of 100 µm or larger in particles constituting the powder or granular carbonic acid foaming composition according to the first embodiment of the present invention can be determined by sieving the particles through a sieve with an opening of 100 µm, SEM-observing the obtained particles having a particle size of 100 µm or larger, and actually measuring the proportion by number thereof.

In general, carbonate particles and organic acid particles serving as starting materials have a high hardness. If the starting materials having a large particle size are used as they are, the content of the 100 µm or larger particles having a hardness of 5 gf or higher is elevated and texture is deteriorated due to particles left undissolved in dissolving the carbonic acid foaming composition in water. However, use of the granulated particles as at least a part of the 100 µm or larger particles can decrease the content of particles having a high hardness, reduces roughness in texture, and can offer favorable feeling in use.

Examples of the case where a part of the particles is granulated particles include the case where either the organic acid or the carbonate is prepared into granulated particles, and the other starting material is used as it is. More specific examples thereof include the case where an additional component, other than the organic acid, constituting the carbonic acid foaming composition, preferably one or more members selected from the group consisting of an excipient, a water-soluble polymer (a binder and a feel modifier), and a moisture absorbent, more preferably all components other than the organic acid, and the carbonate are prepared into granulated particles, and the starting material organic acid is used as it is without being granulated; and the case where an additional component, other than the carbonate, constituting the carbonic acid foaming composition, preferably one or more members selected from the group consisting of an excipient, a water-soluble polymer (a binder and a feel modifier), and a moisture absorbent, more preferably all components other than the carbonate, and the organic acid are prepared into granulated particles, and the starting material carbonate is used as it is without being granulated. It is preferred that the organic acid should be prepared into granulated particles while the carbonate is used as starting material particles, from the viewpoint of production efficiency.

Also preferably, the granulated particles contain the excipient.

The organic acid and the carbonate are preferably each independently in the form of separate particles without being granulated together. The organic acid and the carbonate which are each in the form of independent particles can more improve preservation stability and foamability.

The powder or granular carbonic acid foaming composition according to the first embodiment of the present invention preferably contains particles (A) containing the carbonate and particles (B) containing the organic acid. In this context, preferably, the particles (A) are particles containing no organic acid, and the particles (B) are particles containing no carbonate. The particles (A) or the particles (B) preferably contain the excipient, or both the particles (A) and the particles (B) preferably contain the excipient. Specifically, the powder or granular carbonic acid foaming composition according to the first embodiment of the present invention may contain particles (A) containing the carbonate and particles (B) containing the organic acid and the excipient, may contain particles (A) containing the carbonate and the excipient and particles (B) containing the organic acid, or may contain particles (A) containing the carbonate and the excipient and particles (B) containing the organic acid and the excipient.

The content (proportion by mass) of the particles having a particle size of 100 µm or larger in all particles constituting the powder or granular carbonic acid foaming composition according to the first embodiment of the present invention can be measured by a sieving method.

The content of the particles having a particle size of 100 µm or larger and having a hardness of 5 gf or higher in all particles constituting the powder or granular carbonic acid foaming composition according to the first embodiment of the present invention can be measured by the following method.
(i) First, the content (proportion by number) of the particles having a particle size of 100 µm or larger in all the particles is calculated.

Specifically, a particle size distribution is measured using CAMSIZER XT (particle size measurement apparatus, manufactured by Retsch GmbH), and particle sizes of from 1 to 3 000 µm in the obtained distribution are regarded as "all the particles" according to the present invention and evenly divided into 100 intervals as logarithms. Particle sizes in a range equal to or larger than the interval involving 100 µm are counted as 100 µm or larger. The total number of 100 µm or larger particles is calculated, and the content ratio (proportion by number) of the particles having a particle size of 100 µm or larger in all the particles is calculated.

(ii) Next, the content (proportion by number) of the particles having a hardness of 5 gf or higher in the particles having a particle size of 100 µm or larger is calculated.

Specifically, the powder or granular carbonic acid foaming composition is sieved through a sieve with an opening of 100 µm, and arbitrary 20 or more particles are sampled from the obtained particles having a particle size of 100 µm or larger. The hardnesses of the particles are measured one by one using a micro compression tester (manufactured by Shimadzu Corp. MCT series), and the content ratio of the particles having a hardness of 5 gf or higher is calculated. This operation is repeated three times, and an average value is calculated.

As for the hardnesses of the particles, a load (gf) at a breaking point of each particle loaded using the micro compression tester can be adopted as the hardness of this particle. When a particle collapses at multiple stages, a load at an initial breaking point (hereinafter, also referred to as the first breaking point) is adopted as the hardness of this particle.

(iii) Next, the content (proportion by number) of the particles having a particle size of 100 µm or larger and having a hardness of 5 gf or higher in all the particles can be calculated by multiplying the content (proportion by number) of the particles having a particle size of 100 µm or larger in all the particles obtained in the step (i) by the content (proportion by number) of the particles having a hardness of 5 gf or higher in the particles having a particle size of 100 µm or larger obtained in the step (ii).

The particles having a particle size of 100 µm or larger are contained at 30% by mass or more, preferably 35% by mass or more, more preferably 40% by mass or more, further more preferably 50% by mass or more, even more preferably 55% by mass or more, even more preferably 65% by mass or more, even more preferably 70% by mass or more, in all particles constituting the powder or granular carbonic acid foaming composition according to the first embodiment of the present invention from the viewpoint of preservation stability. The particles having a particle size of 100 µm or larger are contained at preferably 100% by mass or less, more preferably 95% by mass or less, further more preferably 90% by mass or less, from the viewpoint of productivity. The particles having a particle size of 100 µm or larger are contained at 30% by mass or more, preferably 30% by mass or more and 100% by mass or less, more preferably 35% by mass or more and 100% by mass or less, further more preferably 40% by mass or more and 95% by mass or less, even more preferably 50% by mass or more and 90% by mass or less, even more preferably 55% by mass or more and 90% by mass or less, even more preferably 65% by mass or more and 90% by mass or less, even more preferably 70% by mass or more and 90% by mass or less, in all the particles.

The particles having a particle size of 100 µm or larger are of preferably 0.015% by number or more, more preferably 0.017% by number or more, further more preferably 0.02% by number or more, further more preferably 0.03% by number or more, further more preferably 0.1% by number or more, in all particles constituting the powder or granular carbonic acid foaming composition according to the first embodiment of the present invention from the viewpoint of preservation stability. The upper limit value is preferably 1% by number or less, more preferably 0.7% by number or less, further more preferably 0.5% by number or less, even more preferably 0.4% by number or less, from the viewpoint of productivity because a fine powder is also involved in the course of production, though its ideal value is preferably 100% by number. The particles having a particle size of 100 µm or larger are of preferably 0.015% by number or more and 1% by number or less, more preferably 0.017% by number or more and 0.7% by number or less, further more preferably 0.02% by number or more and 0.5% by number or less, further more preferably 0.03% by number or more and 0.5% by number or less, further more preferably 0.1% by number or more and 0.4% by number or less, in all the particles.

The content of the particles having a particle size of 100 µm or larger and having a hardness of 5 gf or higher is 0.10% by number or less, preferably 0.07% by number or less, more preferably 0.05% by number or less, further more preferably 0.04% by number or less, even more preferably 0% by number, in all particles constituting the powder or granular carbonic acid foaming composition according to the first embodiment of the present invention from the viewpoint of improving feeling in use and, particularly, reducing roughness in texture. The lower limit value may be, for example, preferably 0.001% by number or more, more preferably 0.005% by number or more, further more preferably 0.01% by number or more, from the viewpoint of productivity.

The particles having a hardness of 5 gf or higher are of preferably 45% by number or less in the particles having a particle size of 100 µm or larger. When the particles are of 45% by number or less, feeling in use can be improved and, particularly, roughness in texture can be reduced. The particles having a hardness of 5 gf or higher are of preferably 40% by number or less, more preferably 35% by number or less, further more preferably 30% by number or less, in the particles having a particle size of 100 µm or larger. The lower limit value is not particularly limited and is preferably 1% by number or more, more preferably 5% by number or more, further more preferably 10% by number or more, from the viewpoint of productivity, though the particles are preferably absent, i.e., of 0% by number.

The content of a fine powder having a particle size of 10 µm or smaller is preferably 3.5% by mass or less, more preferably 3.3% by mass or less, further more preferably 3.0% by mass or less, even more preferably 2.0% by mass or less, even more preferably 1.0% by mass or less, in all particles constituting the powder or granular carbonic acid foaming composition according to the first embodiment of the present invention from the viewpoint of improving feeling in use and preservation stability and, particularly, preventing choking on the fine powder. The content is preferably 0.001% by mass or more, more preferably 0.002% by mass or more, further more preferably 0.003% by mass or more, from the viewpoint of productivity. The content of the fine powder having a particle size of 10 µm or smaller is preferably 0.001% by mass or more and 3.5% by mass or less, more preferably 0.002% by mass or more and 3.3% by mass or less, further more preferably 0.003% by mass or more and 3.0% by mass or less, even more preferably 0.003% by mass or more and 2.0% by mass or less, even more preferably 0.003% by mass or more and 1.0% by mass or less, in all the particles.

The average hardness value of the particles having a particle size of 100 µm or larger is preferably 0.3 gf or higher, more preferably 0.4 gf or higher, further more preferably 0.5 gf or higher, from the viewpoint of improving preservation stability, and preferably lower than 5 gf, more preferably 4.7 gf or lower, further more preferably 4.5 gf or lower, from the viewpoint of improving feeling in use and, particularly, reducing roughness in texture. The average hardness value of the particles having a particle size of 100 µm or larger is preferably 0.3 gf or higher and lower than 5 gf, more preferably 0.4 gf or higher and 4.7 gf or lower, further more preferably 0.5 gf or higher and 4.5 gf or lower. The average hardness value is determined by sampling arbitrary 20 or more particles from the particles having a particle size of 100 µm or larger, measuring the hardnesses of the particles one by one using a micro compression tester (manufactured by Shimadzu Corp. MCT series), and calculating an average value thereof.

The powder or granular carbonic acid foaming composition according to the first embodiment of the present invention can be suitably used as a foaming aid which helps a surfactant-containing composition, particularly, a hair cosmetic such as a shampoo or a conditioning agent, make lather.

The powder or granular carbonic acid foaming composition according to the first embodiment of the present invention can be enclosed in a packaging material and provided. The powder or granular carbonic acid foaming composition according to the first embodiment of the present invention is excellent in preservation stability in the form of a product enclosed in a packaging material because the composition, even when enclosed in the packaging material and preserved, is unlikely to cause the swelling of the packaging material ascribable to the generation of carbonic acid gas.

The shape of the packaging material is not particularly limited so long as it has a structure where the powder or granular carbonic acid foaming composition can be enclosed. Examples thereof include bag and bottle shapes. Among them, a bag-shaped packaging material is preferred.

A material constituting the packaging material is not particularly limited so long as the powder or granular carbonic acid foaming composition can be enclosed therein. For example, a resin film, or a laminated film of an inorganic thin film of a metal or a metal oxide laminated on a resin film can be used for a bag-shaped packaging material.

### <Method for producing powder or granular carbonic acid foaming composition>

The method for producing the powder or granular carbonic acid foaming composition according to the first embodiment of the present invention contains a step of granulating at least one of a carbonate and an organic acid. One of the starting materials, i.e., the carbonate and the organic acid, may be used as it is without being granulated, or the carbonate and the organic acid may be separately granulated and used as granulated particles of the carbonate and granulated particles of the organic acid, respectively.

By granulating at least either the carbonate or the organic acid, a powder or granular carbonic acid foaming composition having an adjusted particle size and hardness can be produced, and both of the preservation stability and the feeling in use can be attained.

The method for producing the powder or granular carbonic acid foaming composition according to the first embodiment of the present invention preferably contains a step of mixing particles (A) containing the carbonate and particles (B) containing the organic acid.

The particles (A) containing the carbonate preferably contain no organic acid. The particles (A) containing the carbonate may be particles of a starting material itself or may be granulated particles of the carbonate. On the other hand, the particles (B) containing the organic acid preferably contain no carbonate. The particles (B) containing the organic acid may be particles of a starting material itself or may be granulated particles of the organic acid. The particles (A), the particles (B), or both the particles (A) and the particles (B) preferably contain the excipient.

In the case of granulating the carbonate, an additional component, other than the organic acid, constituting the cleansing composition, preferably one or more members selected from the group consisting of a surfactant, an excipient, a binder, and a moisture absorbent, more preferably all components other than the organic acid, and the carbonate are preferably granulated to give granulated particles.

In the case of granulating the organic acid, an additional component, other than the carbonate, constituting the cleansing composition, preferably one or more members selected from the group consisting of a surfactant, an excipient, a binder, and a moisture absorbent, more preferably all components other than the carbonate, and the organic acid are preferably granulated to give granulated particles.

A carbonate and an organic acid having a median diameter of 170 µm or smaller are preferably used as starting materials.

More specifically, the median diameter of the carbonate to be used as a starting material is preferably 10 µm or larger, more preferably 15 µm or larger, further more preferably 20 µm or larger, from the viewpoint of improving productivity and preservation stability. The median diameter is preferably 170 µm or smaller, more preferably 150 µm or smaller, further more preferably 120 µm or smaller, even more preferably 110 µm or smaller, even more preferably 100 µm or smaller, even more preferably 95 µm or smaller, even more preferably 90 µm or smaller, from the viewpoint of attaining favorable foamability and texture. The median diameter of the carbonate to be used as a starting material is preferably 10 µm or larger and 170 µm or smaller, more preferably 10 µm or larger and 150 µm or smaller, further more preferably 15 µm or larger and 120 µm or smaller, even more preferably 20 µm or larger and 110 µm or smaller, even more preferably 20 µm or larger and 100 µm or smaller, even more preferably 20 µm or larger and 95 µm or smaller, even more preferably 20 µm or larger and 90 µm or smaller.

In the case of granulating the carbonate, the median diameter of the carbonate as a starting material is further more preferably in the range described above as well as even more preferably 100 µm or smaller, even more preferably 90 µm or smaller, even more preferably 70 µm or smaller, even more preferably 60 µm or smaller, from the same viewpoint as described above as well as the viewpoint of more improving foamability and texture, and is even more preferably 10 µm or larger and 100 µm or smaller, even more preferably 10 µm or larger and 90 µm or smaller, even more preferably 10 µm or larger and 70 µm or smaller, even more preferably 10 µm or larger and 60 µm or smaller.

In the case of using the starting material carbonate as it is without being granulated, the median diameter is further more preferably in the range described above as well as even more preferably 80 µm or larger, even more preferably 90 µm or larger, and even more preferably 110 µm or smaller, from the same viewpoint as described above as well as the viewpoint of the balance between preservation stability and texture, and is even more preferably 80 µm or larger and 170 µm or smaller, even more preferably 80 µm or larger and 150 µm or smaller, even more preferably 90 µm or larger and 120 µm or smaller, even more preferably 90 µm or larger and 110 µm or smaller.

The median diameter of the organic acid as starting material particles is preferably 10 µm or larger, more preferably 15 µm or larger, further more preferably 20 µm or larger, from the viewpoint of improving productivity and preservation stability. The median diameter is preferably 500 µm or smaller, more preferably 400 µm or smaller, further more preferably 350 µm or smaller, even more preferably 170 µm or smaller, even more preferably 150 µm or smaller, even more preferably 120 µm or smaller, even more preferably 110 µm or smaller, even more preferably 100 µm or smaller, even more preferably 95 µm or smaller, even more preferably 90 µm or smaller, from the viewpoint of attaining favorable foamability and texture. The median diameter of the organic acid as a starting material is preferably 10 µm or larger and 500 µm or smaller, more preferably 10 µm or larger and 400 µm or smaller, further more preferably 10 µm or larger and 350 µm or smaller, preferably 10 µm or larger and 170 µm or smaller, more preferably 10 µm or larger and 150 µm or smaller, further more preferably 15 µm or larger and 120 µm or smaller, even more preferably 20 µm or larger and 110 µm or smaller, even more preferably 20 µm or larger and 100 µm or smaller, even more preferably 20 µm or larger and 95 µm or smaller, even more preferably 20 µm or larger and 90 µm or smaller.

In the case of granulating the organic acid, the median diameter of the organic acid as a starting material is further more preferably in the range described above as well as even more preferably 30 µm or larger, and even more preferably 70 µm or smaller, even more preferably 60 µm or smaller, from the same viewpoint as described above as well as the viewpoint of more improving foamability and texture, and is even more preferably 10 µm or larger and 100 µm or smaller, even more preferably 10 µm or larger and 90 µm or smaller, even more preferably 10 µm or larger and 70 µm or smaller, even more preferably 10 µm or larger and 60 µm or smaller.

In the case of using the starting material organic acid as it is without being granulated, the median diameter is preferably in the range described above as well as even more preferably 80 µm or larger, even more preferably 90 µm or larger, and even more preferably 110 µm or smaller, from the same viewpoint as described above as well as the viewpoint of the balance between preservation stability and texture, and is even more preferably 80 µm or larger and 170 µm or smaller, even more preferably 80 µm or larger and 150 µm or smaller, even more preferably 90 µm or larger and 120 µm or smaller, even more preferably 90 µm or larger and 110 µm or smaller.

The carbonate and the organic acid are preferably produced so as to have the following median diameters, respectively.

The median diameter of the carbonate in the powder or granular carbonic acid foaming composition (product) is preferably 80 µm or larger, more preferably 90 µm or larger, further more preferably 100 µm or larger, even more preferably 110 µm or larger, even more preferably 120 µm or larger, from the viewpoint of preservation stability. The median diameter is preferably 500 µm or smaller, more preferably 400 µm or smaller, further more preferably 300 µm or smaller, from the viewpoint of productivity. The median diameter of a granulation product of the carbonate in the powder or granular carbonic acid foaming composition (product) is preferably 80 µm or larger and 500 µm or smaller, more preferably 90 µm or larger and 400 µm or smaller, further more preferably 100 µm or larger and 300 µm or smaller. The carbonate is preferably prepared into a granulation product from the viewpoint of improving feeling in use and, particularly, reducing roughness in texture. In this case, the median diameter preferably falls within the range described above after granulation of the carbonate and before blending. The median diameter of the granulation product is in the suitable range described above as well as even more preferably 110 µm or larger and 300 µm or smaller, even more preferably 120 µm or larger and 300 µm or smaller, from the viewpoint of more improving preservation stability. In the case of using the starting material carbonate as it is, a preferred range of the median diameter of the carbonate in the powder or granular carbonic acid foaming composition (product) is as described about the median diameter of the carbonate as the starting material particles mentioned above.

The median diameter of the organic acid in the powder or granular carbonic acid foaming composition (product) is preferably 80 µm or larger, more preferably 90 µm or larger, further more preferably 100 µm or larger, even more preferably 110 µm or larger, even more preferably 120 µm or larger, from the viewpoint of preservation stability. The median diameter is preferably 500 µm or smaller, more preferably 400 µm or smaller, further more preferably 300 µm or smaller, from the viewpoint of productivity. The median diameter of a granulation product of the organic acid in the powder or granular carbonic acid foaming composition (product) is preferably 80 µm or larger and 500 µm or smaller, more preferably 90 µm or larger and 400 µm or smaller, further more preferably 100 µm or larger and 300 µm or smaller. The organic acid is preferably prepared into a granulation product from the viewpoint of improving feeling in use and, particularly, reducing roughness in texture. In this case, the median diameter preferably falls within the range described above after granulation of the organic acid and before blending. The median diameter of the granulation product is in the suitable range described above as well as even more preferably 110 µm or larger and 300 µm or smaller, even more preferably 120 µm or larger and 300 µm or smaller, from the viewpoint of more improving preservation stability. In the case of using the starting material organic acid as it is, a preferred range of the median diameter of the organic acid in the powder or granular carbonic acid foaming composition (product) is as described about the median diameter of the organic acid as the starting material particles mentioned above.

In this context, the median diameter (D50) means a particle size at which a cumulative volume frequency calculated from volume fractions is 50% when calculated from smaller toward larger particle sizes. Specifically, the median diameter can be measured by a method described in Examples.

The starting material particles may be granulated after particles having a particle size of preferably 500 µm or larger, more preferably 400 µm or larger, further more preferably 300 µm or larger, are removed therefrom in advance using a sieve, from the viewpoint of attaining more favorable texture.

An additional starting material component used other than the carbonate and the organic acid has a median diameter equal to or smaller than the median diameters of the carbonate and the organic acid, and the median diameter is usually 170 µm or smaller, for example, 5 µm or larger and 150 µm or smaller.

Any of a fluidized-bed granulation method, a stirring granulation method, a tumbling granulation method, and an extrusion granulation method can be used as a granulation method. Among those described above, a fluidized-bed granulation method is preferred from the viewpoint of improving the solubility of the powder or granular carbonic acid foaming composition in a liquid (water).

Specifically, granulation can, for example, be carried out by charging starting material particles into a fluidized-bed granulator, and adding a binder solution of a binder dissolved in water, ethanol, or a mixed liquid thereof to a uniformly mixed powder and granular material. As mentioned above, the carbonate and the organic acid are preferably not contained at the same time in the same granulated particle from the viewpoint of improving preservation stability.

The granulated particles can be mixed with ungranulated particles by a known means to produce a powder or granular carbonic acid foaming composition. In the case of separately granulating the carbonate and the organic acid, the respective granulated particles (granulated particles containing the carbonate and granulated particles containing the organic acid) can be similarly mixed by use of a known means to produce a powder or granular carbonic acid foaming composition.

The binder to be used in granulation in the present invention is preferably selected from the group consisting of the water-soluble polymers described above. One or more members selected from the group consisting of hydroxyethylcellulose, hydroxymethylcellulose, carboxymethylcellulose, xanthan gum, carrageenan, and guar gum can be used from the viewpoint of the instant solubility and productivity of the carbonic acid foaming composition, and one or more members selected from the group consisting of hydroxyethylcellulose, hydroxymethylcellulose, carboxymethylcellulose, xanthan gum, and carrageenan are preferably used.

The content (solid content) of the binder in the binder solution is preferably 0.1% by mass or more, more preferably 0.3% by mass or more, further more preferably 0.5% by mass or more, and preferably 10% by mass or less, more preferably 8% by mass or less, further more preferably 5% by mass or less, from the viewpoint of productivity. The content of the binder in the binder solution is preferably from 0.1 to 10% by mass, more preferably from 0.3 to 8% by mass, further more preferably from 0.5 to 5% by mass.

The content (solid content) of the binder in the carbonic acid foaming composition is preferably 0.01% by mass or more, more preferably 0.05% by mass or more, further more preferably 0.1% by mass or more, and preferably 10% by mass or less, more preferably 5% by mass or less, further more preferably 3% by mass or less, from the viewpoint of instant solubility. The content of the binder in the carbonic acid foaming composition is preferably from 0.01 to 10% by mass, more preferably from 0.05 to 5% by mass, further more preferably from 0.1 to 3% by mass.

### <Method for using powder or granular carbonic acid foaming composition>

The powder or granular carbonic acid foaming composition according to the first embodiment of the present invention is used by bringing the composition into contact with a surfactant-containing composition upon application to form the surfactant-containing composition into a dosage form of foam. More specifically, the powder or granular carbonic acid foaming composition according to the first embodiment of the present invention is brought into contact with a surfactant-containing composition upon application, whereby carbonic acid gas is generated through the reaction between the carbonate and the organic acid contained in the powder or granular carbonic acid foaming composition by a liquid component (water) contained in the surfactant-containing composition or an optionally added liquid component (water), and the surfactant-containing composition can thereby be formed into a dosage form of foam.

The powder or granular carbonic acid foaming composition according to the first embodiment of the present invention starts to foam spontaneously through the reaction with water, and the composition makes quick lather with the surfactant-containing composition and eliminates the need of a special procedure of making lather, which requires time and labor. Thus, the carbonic acid foaming composition according to the first embodiment of the present invention can be conveniently utilized.

The method for using the powder or granular carbonic acid foaming composition according to the first embodiment of the present invention is also a foaming method and a washing method.

Specifically, the method also serves as a foaming method for a surfactant-containing composition, containing bringing the powder or granular carbonic acid foaming composition according to the first embodiment of the present invention into contact with the surfactant-containing composition to form the surfactant-containing composition into a dosage form of foam.

The method also serves as a washing method, containing: bringing the powder or granular carbonic acid foaming composition according to the first embodiment of the present invention into contact with a surfactant-containing composition to form the surfactant-containing composition into a dosage form of foam; and applying the surfactant-containing composition in the form of foam to the surface of the human body for washing.

The method also serves as a hair washing method, containing: bringing the powder or granular carbonic acid foaming composition according to the first embodiment of the present invention into contact with a surfactant-containing composition to form the surfactant-containing composition into a dosage form of foam; and applying the surfactant-containing composition in the form of foam to hair for washing.

In the present specification, the surfactant-containing composition is not particularly limited so long as the composition contains a surfactant and is applicable to the surface of the human body including the skin and hair. Examples thereof include hair cosmetics, face washes, and body cleansers (body shampoos). Examples of the hair cosmetic include shampoos, rinses, conditioning agents, treatment agents, styling agents, hair dyes, and hair growth tonics.

Among those described above, the surfactant-containing composition is preferably a hair cosmetic, more preferably one member selected from the group consisting of a shampoo, a rinse, a conditioning agent, and a treatment agent, further more preferably a shampoo, from the viewpoint of more effectively exerting the advantageous effects of the present invention.

The surfactant-containing composition is also preferably in a liquid state from the viewpoint of being capable of more effectively exerting the advantageous effects of the present invention.

A hair cosmetic, when applied directly to hair, may impose a burden on the hair or the scalp by causing the friction between the hair and the scalp in making lather or spreading over the hair. However, a hair cosmetic used in combination with the powder or granular carbonic acid foaming composition according to the first embodiment of the present invention can be applied in the form of foam, whereby the burden on hair or the scalp can be reduced. Furthermore, the powder or granular carbonic acid foaming composition according to the first embodiment of the present invention is excellent in instant solubility and is therefore also excellent in feeling in use such as feel (texture) even when dissolved in a hair cosmetic. Moreover, the powder or granular carbonic acid foaming composition according to the first embodiment of the present invention is excellent in versatility because the composition can be used in combination with a commercially available hair cosmetic such as a shampoo or a conditioning agent and eliminates the need of a special container or tool for a procedure of making lather. Particularly, the composition enclosed as a portion for one usage in a packaging material can be easy to carry and is therefore also available while on the go. Besides, a blood circulation-promoting effect by carbonic acid gas can also be expected.

The method for using the powder or granular carbonic acid foaming composition according to the first embodiment of the present invention is more specifically performed, for example, by putting the carbonic acid foaming composition on the palm, adding the surfactant-containing composition to the carbonic acid foaming composition, thoroughly blending water into the carbonic acid foaming composition on the palm, then lightly making lather, and applying the foam to a recipient such as the face, hair, or the whole body. The order of putting these compositions on the palm is not limited, and the powder or granular carbonic acid foaming composition is preferably put on the palm first, followed by the addition of the surfactant-containing composition thereto, from the viewpoint of reducing scattering of the powder or granular carbonic acid foaming composition and more effectively causing foaming of the surfactant-containing composition, though the surfactant-containing composition may be put on the palm, followed by the addition of the powder or granular carbonic acid foaming composition to the surfactant-containing composition.

If the foaming of the surfactant-containing composition is unlikely to proceed, water may be further added thereto, if necessary. In this respect, the timing of adding water is not particularly limited, and, for example, the surfactant-containing composition may be added after the powder or granular carbonic acid foaming composition is brought into contact with water, or the powder or granular carbonic acid foaming composition may be added after the surfactant-containing composition is brought into contact with water. Alternatively, water may be added after the powder or granular carbonic acid foaming composition is brought into contact with the surfactant-containing composition. The surfactant composition is preferably added after the powder or granular carbonic acid foaming composition is brought into contact with water from the viewpoint of more effectively causing foaming of the surfactant-containing composition. However, use without the addition of water is preferred from the viewpoint of improvement in feeling in use.

When the surfactant-containing composition is in a liquid state, the amount of the liquid surfactant-containing composition added to the powder or granular carbonic acid foaming composition is preferably 1/30 or more, more preferably 1/25 or more, further more preferably 1/20 or more, and preferably 1/0.5 or less, more preferably 1/1 or less, further more preferably 1/2 or less, in terms of the mass ratio between the powder or granular carbonic acid foaming composition and the surfactant-containing composition to be added (powder or granular carbonic acid foaming composition/surfactant-containing composition) from the viewpoint of improving the solubility of the powder or granular carbonic acid foaming composition and also improving foamability.

The amount of the water added, if necessary, is preferably 0.1 g or more and 20 g or less, more preferably 0.5 g or more and 15 g or less, further more preferably 1 g or more and 10 g or less, per g of the powder or granular carbonic acid foaming composition from the viewpoint of solubility and foamability.

When the surfactant-containing composition is in a liquid state, the powder or granular carbonic acid foaming composition according to the first embodiment of the present invention is applied to an object such as hair within a time of preferably 5 minutes, more preferably 3 minutes, further more preferably 2 minutes, even more preferably 1.5 minutes, after contact with the liquid surfactant-containing composition (or after contact of the powder or granular carbonic acid foaming composition with water when the powder or granular carbonic acid foaming composition is brought into contact with water first) from the viewpoint of the retention of foam.

The temperature of the water to be added, if necessary, to the powder or granular carbonic acid foaming composition is preferably 15°C or higher and 50°C or lower, more preferably 15°C or higher and 45°C or lower, further more preferably 20°C or higher and 45°C or lower, though it is not particularly limited.

### <Hair cosmetic kit>

The hair cosmetic kit according to the first embodiment of the present invention contains a powder or granular carbonic acid foaming composition and a surfactant-containing composition.

The powder or granular carbonic acid foaming composition and the surfactant-containing composition to be used in the hair cosmetic kit according to the first embodiment of the present invention are the same as those mentioned above. The hair cosmetic kit is used by bringing the powder or granular carbonic acid foaming composition into contact with the surfactant-containing composition upon application to form the surfactant-containing composition into a dosage form of foam.

### [Second embodiment: powder or granular cleansing composition]

The powder or granular cleansing composition according to the second embodiment of the present invention contains
a carbonate, an organic acid, and a surfactant, wherein
particles having a particle size of 100 µm or larger are contained at 30% by mass or more, and
a content of particles having a particle size of 100 µm or larger and having a hardness of 5 gf or higher is 0.10% by number or less.

The powder or granular cleansing composition (hereinafter, also simply referred to as the cleansing composition) according to the second embodiment of the present invention can exert the washing effect of the surfactant as well as excellent washing performance by generating carbonic acid gas through the reaction between the carbonate and the organic acid when the cleansing composition is dissolved in water.

The powder or granular cleansing composition according to the second embodiment of the present invention is typically used for washing the face, the body, or the like. Such a cleansing composition to be applied directly to the skin such as the face or the body is required to offer favorable feeling in use without roughness in texture in making lather, and needs to be instantly dissolved in a short time. Thus, particles constituting the cleansing composition desirably have a small particle size. However, if the cleansing composition has a small particle size, a moisture absorbent added thereto has been found to be insufficiently effective, be unable to suppress the generation of carbonic acid gas in a packaging material, and render preservation stability poor. Accordingly, in order to suppress the generation of carbonic acid gas during preservation, the carbonate or the organic acid needs to have a large particle size. As a result of conducting diligent studies to solve these conflicting demands, the present inventors completed the present invention by finding that the hardness and the particle size of particles constituting the cleansing composition are deeply involved in texture upon application and preservation stability, and finding that both the texture and the preservation stability can be achieved by adjusting the hardness or the particle size of the particles in the cleansing composition.

The powder or granular cleansing composition according to the second embodiment of the present invention contains a predetermined amount or more of particles having a particle size of 100 µm or larger and has a predetermined percent by number or less of the content of particles having a particle size of 100 µm or larger and having a hardness of 5 gf or higher, whereby the roughness in texture can be reduced, and good feeling in use can be produced.

### <Carbonate>

The powder or granular cleansing composition according to the second embodiment of the present invention contains a carbonate.

The carbonate to be used in the cleansing composition according to the second embodiment of the present invention is as described in the first embodiment, and its preferred range is also the same thereas.

The content of the carbonate in the powder or granular cleansing composition is preferably 10% by mass or more, more preferably 15% by mass or more, further more preferably 20% by mass or more, even more preferably 30% by mass or more, from the viewpoint of improvement in foamability. The content is preferably 60% by mass or less, more preferably 55% by mass or less, further more preferably 50% by mass or less, from the viewpoint of improvement in long-lasting foam. The content of the carbonate in the powder or granular cleansing composition is preferably 10% by mass or more and 60% by mass or less, more preferably 15% by mass or more and 55% by mass or less, further more preferably 20% by mass or more and 50% by mass or less, even more preferably 30% by mass or more and 50% by mass or less.

The carbonate used in the present invention may be contained as particles of a starting material itself (hereinafter, also referred to as "starting material particles") in the powder or granular cleansing composition according to the second embodiment of the present invention, or may be contained as particles granulated (hereinafter, also referred to as "granulated particles"), if necessary, with an additional component, or may be contained as a mixture thereof. When the carbonate is granulated particles, the carbonate may be granulated alone or may be granulated together with a component other than the carbonate. The granulated particles preferably contain no organic acid from the viewpoint of preservation stability. In this context, the phrase "contain no" means being substantially free thereof, and the amount of the organic acid in the granulated particles is preferably less than 1% by mass, more preferably 0% by mass. The carbonate is preferably granulated together with an additional component other than the organic acid, for example, one or more members selected from the group consisting of a surfactant, an excipient, a binder, and a moisture absorbent, and particularly preferably granulated with all components other than the organic acid. The granulation together with a component other than the organic acid can reduce the amount of a fine powder derived from the component other than the organic acid, and can prevent choking caused by scattered fine powder upon application of the cleansing composition.

### <Organic acid>

The powder or granular cleansing composition according to the second embodiment of the present invention contains an organic acid.

The organic acid used in the present invention is as described in the first embodiment, and its preferred range is also the same thereas.

The content of the organic acid in the powder or granular cleansing composition is preferably 5% by mass or more, more preferably 7% by mass or more, further more preferably 10% by mass or more, from the viewpoint of improvement in foamability. The content is preferably 60% by mass or less, more preferably 50% by mass or less, further more preferably 40% by mass or less, even more preferably 30% by mass or less, even more preferably 20% by mass or less, from the viewpoint of improvement in long-lasting foam. The content of the organic acid in the powder or granular cleansing composition is preferably 5% by mass or more and 60% by mass or less, more preferably 5% by mass or more and 50% by mass or less, further more preferably 7% by mass or more and 40% by mass or less, even more preferably 7% by mass or more and 30% by mass or less, even more preferably 10% by mass or more and 20% by mass or less.

The organic acid used in the present invention may be contained as particles of a starting material itself (starting material particles) in the powder or granular cleansing composition according to the second embodiment of the present invention, or may be contained as particles granulated (granulated particles), if necessary, with an additional component, or may be contained as a mixture thereof. When the organic acid is granulated particles, the organic acid may be granulated alone or may be granulated together with a component other than the organic acid. The granulated particles preferably contain no carbonate from the viewpoint of preservation stability. In this context, the phrase "contain no" means being substantially free thereof, and the amount of the carbonate in the granulated particles is preferably less than 1% by mass, more preferably 0% by mass. The organic acid is preferably granulated together with a component other than the carbonate, for example, one or more members selected from the group consisting of a surfactant, an excipient, a binder, and a moisture absorbent, and particularly preferably granulated with all components other than the carbonate. The granulation together with a component other than the carbonate can reduce the amount of a fine powder derived from the component other than the carbonate, and can prevent choking caused by scattered fine powder upon application of the cleansing composition.

### <Surfactant>

The cleansing composition according to the second embodiment of the present invention contains a surfactant.

Examples of the surfactant to be used in the cleansing composition according to the second embodiment of the present invention include anionic surfactants, cationic surfactants, amphoteric surfactants, and nonionic surfactants. One or more of these surfactants can be used. Hereinafter, their respective specific examples will be shown.

### [Anionic surfactant]

Specific examples of the anionic surfactant include N-acylamino acid salts, N-acyl-N-methylamino acid salts, fatty acid salts, ester salts with fatty acids having 5 or more and 18 or less carbon atoms and isethionic acid, alkyl or alkenyl sulfonates having 10 or more and 18 or less carbon atoms, polyoxyalkylene alkyl ether sulfuric acid ester salts, and linear alkyl benzenesulfonates.

Examples of the counterion of an anionic group in the anionic surfactant include: alkali metal ions such as sodium ions and potassium ions; alkaline earth metal ions such as calcium ions and magnesium ions; ammonium ions; and alkanol ammonium having one to three alkanol groups having 2 or 3 carbon atoms (e.g., monoethanol ammonium, diethanol ammonium, triethanol ammonium, and triisopropanol ammonium). Sodium ions or potassium ions are preferred, and sodium ions are more preferred.

### [Cationic surfactant]

Examples of the cationic surfactant include quaternary ammonium salts such as alkyl trimethyl ammonium salts, alkoxyalkyl trimethyl ammonium salts, dialkyl dimethyl ammonium salts, alkylamide alkyl trimethyl ammonium salts, benzalkonium chloride, and alkylpyridinium salts.

Examples of the counterion of a cationic group in the cationic surfactant include alkyl sulfate ions having 1 or more and 3 or less carbon atoms, sulfate ions, phosphate ions, carboxylate ions having 1 or more and 3 or less carbon atoms (formate ions, acetate ions, and propionate ions), and halide ions such as chloride ions and bromide ions. Among them, halide ions are preferred, and chloride ions are more preferred, from the viewpoint of easy production and easy availability of a starting material.

### [Amphoteric surfactant]

Examples of the amphoteric surfactant include one or more members selected from the group consisting of alkylamine oxides having an alkyl group having 10 or more and 18 or less carbon atoms, and alkyl betaines having an alkyl group having 10 or more and 18 or less carbon atoms.

### [Nonionic surfactant]

Specific examples of the nonionic surfactant include one or more members selected from the group consisting of polyoxyethylene alkyl ethers, polyoxyethylene alkenyl ethers, polyoxyethylene sorbitan fatty acid esters, polyoxyethylene sorbitol fatty acid esters, polyoxyethylene fatty acid esters, alkyl glucosides, alkylalkanolamides, alkyl glyceryl ethers, higher fatty acid sucrose esters, polyglycerin fatty acid esters, polyoxyethylene hydrogenated castor oil, and alkyl saccharides.

Among those described above, an anionic surfactant is preferred, and an ester salt of a fatty acid having 5 or more and 18 or less carbon atoms and isethionic acid is more preferred, from the viewpoint of favorable solubility in water, good lather, and the like. An amino acid-based anionic surfactant such as an N-acylamino acid salt or an N-acyl-N-methylamino acid salt is preferred from the viewpoint of low irritation.

The content of the surfactant in the powder or granular cleansing composition is preferably 10% by mass or more, more preferably 15% by mass or more, further more preferably 20% by mass or more, from the viewpoint of improvement in foamability. The content is preferably 50% by mass or less, more preferably 45% by mass or less, further more preferably 40% by mass or less, even more preferably 35% by mass or less, from the viewpoint of improvement in foam break. The content of the surfactant in the cleansing composition is preferably 10% by mass or more and 50% by mass or less, more preferably 15% by mass or more and 45% by mass or less, further more preferably 15% by mass or more and 40% by mass or less, even more preferably 20% by mass or more and 35% by mass or less.

In the powder or granular cleansing composition, the mass ratio of the total amount of the carbonate and the organic acid to the surfactant [(carbonate + organic acid)/surfactant] is preferably 0.1 or more, more preferably 1.0 or more, further more preferably 1.5 or more, from the viewpoint of foamability, and preferably 20 or less, more preferably 10 or less, further more preferably 5 or less, from the viewpoint of improvement in washing performance. The mass ratio of the total amount of the carbonate and the organic acid [(carbonate + organic acid)/surfactant] is preferably 0.1 or more and 20 or less, more preferably 1.0 or more and 10 or less, further more preferably 1.5 or more and 5 or less.

In the powder or granular cleansing composition, the mass ratio of the organic acid to the carbonate [organic acid/carbonate] is preferably 0.05 or more, more preferably 0.1 or more, further more preferably 0.2 or more, and preferably 10 or less, more preferably 5.0 or less, further more preferably 1.0 or less, from the viewpoint of foamability. The mass ratio of the organic acid to the carbonate [organic acid/carbonate] is preferably 0.05 or more and 10 or less, more preferably 0.1 or more and 5.0 or less, further more preferably 0.2 or more and 1.0 or less.

The total amount of the carbonate and the organic acid in the powder or granular cleansing composition is preferably 35% by mass or more, more preferably 40% by mass or more, further more preferably 45% by mass or more, from the viewpoint of foamability, and preferably 70% by mass or less, more preferably 65% by mass or less, further more preferably 60% by mass or less, from the viewpoint of improvement in long-lasting foam. The total amount of the carbonate and the organic acid in the powder or granular cleansing composition is preferably 35% by mass or more and 70% by mass or less, more preferably 40% by mass or more and 65% by mass or less, further more preferably 45% by mass or more and 60% by mass or less.

The total amount of the carbonate, the organic acid, and the surfactant in the powder or granular cleansing composition is preferably 50% by mass or more, more preferably 60% by mass or more, further more preferably 70% by mass or more, from the viewpoint of improvement in washing performance and foamability, and preferably 95% by mass or less, more preferably 90% by mass or less, further more preferably 85% by mass or less, from the viewpoint of improvement in long-lasting foam. The total amount of the carbonate, the organic acid, and the surfactant in the powder or granular cleansing composition is preferably 50% by mass or more and 95% by mass or less, more preferably 60% by mass or more and 90% by mass or less, further more preferably 70% by mass or more and 85% by mass or less.

### <Moisture absorbent>

The powder or granular cleansing composition according to the second embodiment of the present invention preferably further contains a moisture absorbent.

The moisture absorbent used in the present invention is as described in the first embodiment, and its preferred range is also the same thereas.

The content of the moisture absorbent in the powder or granular cleansing composition is preferably 0.01% by mass or more, more preferably 0.1% by mass or more, further more preferably 1% by mass or more, even more preferably 2% by mass or more, even more preferably 4% by mass or more, from the viewpoint of improvement in preservation stability. The content is preferably 10% by mass or less, more preferably 9% by mass or less, further more preferably 8% by mass or less, from the viewpoint of improvement in foamability. The content of the moisture absorbent in the powder or granular cleansing composition is preferably 0.01% by mass or more and 10% by mass or less, more preferably 0.1% by mass or more and 9% by mass or less, further more preferably 1% by mass or more and 8% by mass or less, even more preferably 2% by mass or more and 8% by mass or less, even more preferably 4% by mass or more and 8% by mass or less.

### <Other components>

The powder or granular cleansing composition according to the second embodiment of the present invention may contain components which are generally used in powder or granular cleansing compositions, so long as the object of the present invention is not impaired. Examples of such other components include excipients, binders, natural dyes, moisturizers, anti-inflammatory agents, germicides, antiperspirants, antioxidants, fragrances, and mixtures thereof.

The excipient used in the present invention is as described in the first embodiment, and its preferred range is also the same thereas.

The content of the excipient in the cleansing composition is preferably 5% by mass or more, more preferably 10% by mass or more, and preferably 50% by mass or less, more preferably 40% by mass or less, further more preferably 30% by mass or less, from the viewpoint of solubility, granulability, foamability, and preservation stability. The content of the excipient in the cleansing composition is preferably 5% by mass or more and 50% by mass or less, more preferably 10% by mass or more and 40% by mass or less, further more preferably 10% by mass or more and 30% by mass or less.

Examples of the binder used in the present invention include hydroxyethylcellulose, hydroxymethylcellulose, carboxymethylcellulose, xanthan gum, and carrageenan. One or more of these binders can be used. The content of the binder in the powder or granular cleansing composition is preferably 0.1% by mass or more, more preferably 0.3% by mass or more, further more preferably 0.5% by mass or more, and preferably 10% by mass or less, more preferably 5% by mass or less, further more preferably 3% by mass or less, from the viewpoint of instant solubility and productivity. The content of the binder in the powder or granular cleansing composition is preferably 0.1% by mass or more and 10% by mass or less, more preferably 0.3% by mass or more and 5% by mass or less, further more preferably 0.5% by mass or more and 3% by mass or less.

### <Powder or granular cleansing composition>

In the powder or granular cleansing composition according to the second embodiment of the present invention, particles having a particle size of 100 µm or larger are contained at 30% by mass or more, and the content of particles having a particle size of 100 µm or larger and having a hardness of 5 gf or higher is 0.10% by number or less.

Specifically, in the powder or granular cleansing composition according to the second embodiment of the present invention, the particles having a particle size of 100 µm or larger are contained at 30% by mass or more in particles constituting the powder or granular cleansing composition, and the content of particles having a particle size of 100 µm or larger and having a hardness of 5 gf or higher is 0.10% by number or less in particles constituting the powder or granular cleansing composition.

The particles having a particle size of 100 µm or larger are contained at 30% by mass or more, whereby preservation stability can be enhanced. The content of particles having a particle size of 100 µm or larger and having a hardness of 5 gf or higher is set to 0.10% by number or less, whereby roughness in texture is reduced, and feeling in use can be rendered favorable.

At least a part of the particles having a particle size of 100 µm or larger is preferably granulated particles. The content of the granulated particles in the particles having a particle size of 100 µm or larger is preferably more than 55% by number, more preferably more than 60% by number, further more preferably more than 65% by number, even more preferably more than 70% by number, even more preferably , all of the particles are the granulated particles, from the viewpoint of improving feeling in use and, particularly, reducing roughness in texture, though particles of a starting material itself may be partially contained from the viewpoint of production efficiency. Since the hardness of the granulated particles can be easily adjusted, the content of the 100 µm or larger particles having a hardness of 5 gf or higher can be adjusted.

The content of the granulated particles in the particles having a particle size of 100 µm or larger in particles constituting the powder or granular cleansing composition according to the second embodiment of the present invention can be determined by sieving the particles through a sieve with an opening of 100 µm, SEM-observing the obtained particles having a particle size of 100 µm or larger, and actually measuring the proportion by number thereof.

In general, carbonate particles and organic acid particles as starting materials have a high hardness. If the starting materials having a large particle size are used as they are, the content of the 100 µm or larger particles having a hardness of 5 gf or higher tends to be high, and texture is deteriorated due to particles left undissolved in dissolving the cleansing composition in water. However, by granulating at least a part of the 100 µm or larger particles, the content of particles having a high hardness can be decreased, the roughness in texture can be reduced, and feeling in use can be improved.

Examples of the case where a part of the particles is granulated particles include the case where any one of the organic acid and the carbonate is prepared into granulated particles, and the other starting material is used as it is. More specific examples thereof include the case where an additional component, other than the organic acid, constituting the cleansing composition, preferably one or more members selected from the group consisting of a surfactant, an excipient, a binder, and a moisture absorbent, more preferably all components other than the organic acid, and the carbonate are prepared into granulated particles, and the starting material organic acid is used as it is without being granulated; and the case where an additional component, other than the carbonate, constituting the cleansing composition, preferably one or more members selected from the group consisting of a surfactant, an excipient, a binder, and a moisture absorbent, more preferably all components other than the carbonate, and the organic acid are prepared into granulated particles, and the starting material carbonate is used as it is without being granulated. It is preferred that the organic acid should be prepared into granulated particles while the carbonate is used as starting material particles, from the viewpoint of production efficiency. Also preferably, the granulated particles contain the surfactant.

The organic acid and the carbonate are preferably each independently in the form of separate particles without granulating the organic acid and the carbonate together. The organic acid and the carbonate which are each in the form of independent particles can more improve preservation stability and foamability.

The powder or granular cleansing composition according to the second embodiment of the present invention preferably contains particles (A) containing the carbonate and particles (B) containing the organic acid. In this context, preferably, the particles (A) are particles containing no organic acid, and the particles (B) are particles containing no carbonate. The particles (A), the particles (B) preferably contain the surfactant, or both the particles (A) and the particles (B) preferably contain the surfactant. Specifically, the powder or granular cleansing composition according to the second embodiment of the present invention may contain particles (A) containing the carbonate and particles (B) containing the organic acid and the surfactant, may contain particles (A) containing the carbonate and the surfactant and particles (B) containing the organic acid, or may contain particles (A) containing the carbonate and the surfactant and particles (B) containing the organic acid and the surfactant.

The content (proportion by mass) of the particles having a particle size of 100 µm or larger in all particles constituting the powder or granular cleansing composition according to the second embodiment of the present invention can be measured by a sieving method.

The content of the particles having a particle size of 100 µm or larger and having a hardness of 5 gf or higher in all particles constituting the powder or granular cleansing composition according to the second embodiment of the present invention can be measured by the following method.
(i) First, the content (proportion by number) of the particles having a particle size of 100 µm or larger in all the particles is calculated.

Specifically, a particle size distribution is measured using CAMSIZER XT (particle size measurement apparatus, manufactured by Retsch GmbH), and particle sizes of from 1 to 3 000 µm in the obtained distribution are regarded as "all the particles" according to the present invention and evenly divided into 100 intervals as logarithms. Particle sizes in a range equal to or larger than the interval involving 100 µm are counted as 100 µm or larger. The total number of 100 µm or larger particles is calculated, and the content ratio (proportion by number) of the particles having a particle size of 100 µm or larger in all the particles is calculated.

(ii) Next, the content (proportion by number) of the particles having a hardness of 5 gf or higher in the particles having a particle size of 100 µm or larger is calculated.

Specifically, the powder or granular cleansing composition is sieved through a sieve with an opening of 100 µm, and arbitrary 20 or more particles are sampled from the obtained particles having a particle size of 100 µm or larger. The hardnesses of the particles are measured one by one using a micro compression tester (manufactured by Shimadzu Corp. MCT series), and the content ratio of the particles having a hardness of 5 gf or higher is calculated. This operation is repeated three times, and an average value is calculated.

As for the hardnesses of the particles, a load (gf) at a breaking point of each particle loaded using the micro compression tester can be adopted as the hardness of this particle. When a particle collapses at multiple stages, a load at an initial breaking point (hereinafter, also referred to as the first breaking point) is adopted as the hardness of this particle.

(iii) Next, the content (proportion by number) of the particles having a particle size of 100 µm or larger and having a hardness of 5 gf or higher in all the particles can be calculated by multiplying the content (proportion by number) of the particles having a particle size of 100 µm or larger in all the particles obtained in the step (i) by the content (proportion by number) of the particles having a hardness of 5 gf or higher in the particles having a particle size of 100 µm or larger obtained in the step (ii).

The particles having a particle size of 100 µm or larger are contained at 30% by mass or more, preferably 35% by mass or more, more preferably 40% by mass or more, further more preferably 50% by mass or more, even more preferably 55% by mass or more, even more preferably 65% by mass or more, even more preferably 70% by mass or more, in all particles constituting the powder or granular cleansing composition according to the second embodiment of the present invention from the viewpoint of preservation stability. The particles having a particle size of 100 µm or larger are contained at preferably 100% by mass or less, more preferably 95% by mass or less, further more preferably 90% by mass or less, from the viewpoint of productivity. The particles having a particle size of 100 µm or larger are contained at 30% by mass or more, preferably 30% by mass or more and 100% by mass or less, more preferably 35% by mass or more and 100% by mass or less, further more preferably 40% by mass or more and 95% by mass or less, even more preferably 50% by mass or more and 90% by mass or less, even more preferably 55% by mass or more and 90% by mass or less, even more preferably 65% by mass or more and 90% by mass or less, even more preferably 70% by mass or more and 90% by mass or less, in all the particles.

The particles having a particle size of 100 µm or larger are of preferably 0.015% by number or more, more preferably 0.017% by number or more, further more preferably 0.02% by number or more, further more preferably 0.03% by number or more, further more preferably 0.1% by number or more, in all particles constituting the powder or granular cleansing composition according to the second embodiment of the present invention from the viewpoint of preservation stability. The upper limit value is preferably 1% by number or less, more preferably 0.7% by number or less, further more preferably 0.5% by number or less, from the viewpoint of productivity because a fine powder is also involved in the production process, though its ideal value is preferably 100% by number. The particles having a particle size of 100 µm or larger are of preferably 0.015% by number or more and 1% by number or less, more preferably 0.017% by number or more and 0.7% by number or less, further more preferably 0.02% by number or more and 0.5% by number or less, further more preferably 0.03% by number or more and 0.5% by number or less, further more preferably 0.1% by number or more and 0.5% by number or less, in all the particles.

The content of the particles having a particle size of 100 µm or larger and having a hardness of 5 gf or higher is 0.10% by number or less, preferably 0.07% by number or less, more preferably 0.05% by number or less, further more preferably 0.04% by number or less, even more preferably 0% by number, in all particles constituting the powder or granular cleansing composition according to the second embodiment of the present invention from the viewpoint of improving feeling in use and, particularly, reducing roughness in texture. The lower limit value may be, for example, preferably 0.001% by number or more, more preferably 0.005% by number or more, further more preferably 0.01% by number or more, from the viewpoint of productivity.

The particles having a hardness of 5 gf or higher are of preferably 45% by number or less in the particles having a particle size of 100 µm or larger. When the particles are of 45% by number or less, feeling in use can be improved and, particularly, roughness in texture can be reduced. The particles having a hardness of 5 gf or higher are of preferably 40% by number or less, more preferably 35% by number or less, further more preferably 30% by number or less, even more preferably 25% by number or less, in the particles having a particle size of 100 µm or larger. The lower limit value is not particularly limited and is preferably 1% by number or more, more preferably 5% by number or more, further more preferably 10% by number or more, from the viewpoint of productivity, though the particles are preferably absent, i.e., of 0% by number.

The content of a fine powder having a particle size of 10 µm or smaller is preferably 3.5% by mass or less, more preferably 3.3% by mass or less, further more preferably 3.0% by mass or less, even more preferably 2.0% by mass or less, even more preferably 1.0% by mass or less, in all particles constituting the powder or granular cleansing composition according to the second embodiment of the present invention from the viewpoint of improving feeling in use and preservation stability and, particularly, preventing choking on the fine powder. The content is preferably 0.001% by mass or more, more preferably 0.002% by mass or more, further more preferably 0.003% by mass or more, from the viewpoint of productivity. The content of the fine powder having a particle size of 10 µm or smaller is preferably 0.001% by mass or more and 3.5% by mass or less, more preferably 0.002% by mass or more and 3.3% by mass or less, further more preferably 0.003% by mass or more and 3.0% by mass or less, even more preferably 0.003% by mass or more and 2.0% by mass or less, even more preferably 0.003% by mass or more and 1.0% by mass or less, in all the particles.

The average hardness value of the particles having a particle size of 100 µm or larger is preferably 0.3 gf or higher, more preferably 0.4 gf or higher, further more preferably 0.5 gf or higher, from the viewpoint of improving preservation stability, and preferably lower than 5 gf, more preferably 4.7 gf or lower, further more preferably 4.5 gf or lower, from the viewpoint of improving feeling in use and, particularly, reducing roughness in texture. The average hardness value is determined by sampling arbitrary 20 or more particles from the particles having a particle size of 100 µm or larger, measuring the hardnesses of the particles one by one using a micro compression tester (manufactured by Shimadzu Corp. MCT series), and calculating an average value thereof.

The powder or granular cleansing composition according to the second embodiment of the present invention can be suitably used in a face care product such as a face wash or a body care product such as a hand soap or a body soap.

A foamable cleansing product prepared using the powder or granular cleansing composition according to the second embodiment of the present invention can be enclosed in a packaging material and provided. The powder or granular cleansing composition according to the second embodiment of the present invention is excellent in preservation stability in the form of a product enclosed in a packaging material because the composition, even when enclosed and preserved in the packaging material, is unlikely to cause the swelling of the packaging material ascribable to the generation of carbonic acid gas.

The shape of the packaging material is not particularly limited so long as it has a structure where the powder or granular cleansing composition can be enclosed. Examples thereof include bag and bottle shapes. Among them, a bag-shaped packaging material is preferred.

A material constituting the packaging material is not particularly limited so long as the powder or granular cleansing composition can be enclosed therein. For example, a resin film, or a laminated film of an inorganic thin film of a metal or a metal oxide laminated on a resin film can be used for a bag-shaped packaging material.

### <Method for producing powder or granular cleansing composition>

The method for producing the powder or granular cleansing composition according to the second embodiment of the present invention contains a step of granulating at least one of a carbonate and an organic acid. One of the starting materials, i.e., the carbonate and the organic acid, may be used as it is without being granulated, or the carbonate and the organic acid may be separately granulated and used as granulated particles of the carbonate and granulated particles of the organic acid, respectively.

By granulating at least either the carbonate or the organic acid, a cleansing composition having an adjusted particle size and hardness can be produced, and both of the preservation stability and the feeling in use can be attained.

The method for producing the powder or granular cleansing composition according to the second embodiment of the present invention preferably contains a step of mixing particles (A) containing the carbonate and particles (B) containing the organic acid.

The particles (A) containing the carbonate preferably contain no organic acid. The particles (A) containing the carbonate may be particles of a starting material itself or may be granulated particles of the carbonate. On the other hand, the particles (B) containing the organic acid preferably contain no carbonate. The particles (B) containing the organic acid may be particles of a starting material itself or may be granulated particles of the organic acid. The particles (A), the particles (B), or both the particles (A) and the particles (B) preferably contain the surfactant.

In the case of granulating the carbonate, an additional component, other than the organic acid, constituting the cleansing composition, preferably one or more members selected from the group consisting of a surfactant, an excipient, a binder, and a moisture absorbent, more preferably all components other than the organic acid, and the carbonate are preferably granulated to give granulated particles.

In the case of granulating the organic acid, an additional component, other than the carbonate, constituting the cleansing composition, preferably one or more members selected from the group consisting of a surfactant, an excipient, a binder, and a moisture absorbent, more preferably all components other than the carbonate, and the organic acid are preferably granulated to give granulated particles.

A carbonate and an organic acid having a median diameter of 170 µm or smaller are preferably used as starting materials.

More specifically, the median diameter of the carbonate to be used as a starting material is preferably 10 µm or larger, more preferably 15 µm or larger, further more preferably 20 µm or larger, from the viewpoint of improving productivity and preservation stability. The median diameter is preferably 170 µm or smaller, more preferably 150 µm or smaller, further more preferably 120 µm or smaller, even more preferably 110 µm or smaller, even more preferably 100 µm or smaller, even more preferably 95 µm or smaller, even more preferably 90 µm or smaller, from the viewpoint of attaining favorable texture. The median diameter of the carbonate to be used as a starting material is preferably 10 µm or larger and 170 µm or smaller, more preferably 15 µm or larger and 150 µm or smaller, further more preferably 20 µm or larger and 120 µm or smaller, even more preferably 20 µm or larger and 110 µm or smaller, even more preferably 20 µm or larger and 100 µm or smaller, even more preferably 20 µm or larger and 95 µm or smaller, even more preferably 20 µm or larger and 90 µm or smaller.

In the case of granulating the carbonate, the median diameter of the carbonate as a starting material is further more preferably in the range described above as well as even more preferably 100 µm or smaller, even more preferably 90 µm or smaller, even more preferably 70 µm or smaller, even more preferably 60 µm or smaller, from the same viewpoint as described above and the viewpoint of more improving texture, and is even more preferably 10 µm or larger and 100 µm or smaller, even more preferably 10 µm or larger and 90 µm or smaller, even more preferably 10 µm or larger and 70 µm or smaller, even more preferably 10 µm or larger and 60 µm or smaller.

In the case of using the starting material carbonate as it is without being granulated, the median diameter is further more preferably in the range described above as well as even more preferably 80 µm or larger, even more preferably 90 µm or larger, and even more preferably 110 µm or smaller, from the same viewpoint as described above as well as the viewpoint of the balance between preservation stability and texture, and is even more preferably 80 µm or larger and 170 µm or smaller, even more preferably 80 µm or larger and 150 µm or smaller, even more preferably 90 µm or larger and 120 µm or smaller, even more preferably 90 µm or larger and 110 µm or smaller.

The median diameter of the organic acid as starting material particles is preferably 10 µm or larger, more preferably 15 µm or larger, further more preferably 20 µm or larger, from the viewpoint of improving productivity and preservation stability. The median diameter is preferably 170 µm or smaller, more preferably 150 µm or smaller, further more preferably 120 µm or smaller, further more preferably 110 µm or smaller, even more preferably 100 µm or smaller, further more preferably 95 µm or smaller, even more preferably 90 µm or smaller, even more preferably 80 µm or smaller, from the viewpoint of attaining favorable texture. The median diameter of the organic acid as a starting material is preferably 10 µm or larger and 170 µm or smaller, more preferably 15 µm or larger and 150 µm or smaller, further more preferably 20 µm or larger and 120 µm or smaller, even more preferably 20 µm or larger and 110 µm or smaller, even more preferably 20 µm or larger and 100 µm or smaller, even more preferably 20 µm or larger and 95 µm or smaller, even more preferably 20 µm or larger and 90 µm or smaller, even more preferably 20 µm or larger and 80 µm or smaller.

In the case of granulating the organic acid, the median diameter of the organic acid as a starting material is further more preferably in the range described above as well as even more preferably 30 µm or larger, and even more preferably 70 µm or smaller, even more preferably 60 µm or smaller, even more preferably 50 µm or smaller, from the same viewpoint as described above and the viewpoint of more improving texture, and is even more preferably 20 µm or larger and 70 µm or smaller, even more preferably 20 µm or larger and 60 µm or smaller, even more preferably 20 µm or larger and 50 µm or smaller.

In the case of using the starting material organic acid as it is without being granulated, the median diameter is preferably in the range described above as well as even more preferably 80 µm or larger, even more preferably 90 µm or larger, and even more preferably 110 µm or smaller, from the same viewpoint as described above as well as the viewpoint of the balance between preservation stability and texture, and is even more preferably 80 µm or larger and 170 µm or smaller, even more preferably 80 µm or larger and 150 µm or smaller, even more preferably 90 µm or larger and 120 µm or smaller, even more preferably 90 µm or larger and 110 µm or smaller.

The carbonate and the organic acid are preferably produced so as to have the following median diameters, respectively.

The median diameter of the carbonate in the powder or granular cleansing composition (product) is preferably 80 µm or larger, more preferably 90 µm or larger, further more preferably 100 µm or larger, even more preferably 110 µm or larger, even more preferably 120 µm or larger, from the viewpoint of preservation stability. The median diameter is preferably 500 µm or smaller, more preferably 400 µm or smaller, further more preferably 300 µm or smaller, even more preferably 200 µm or smaller, from the viewpoint of productivity. The median diameter of a granulation product of the carbonate in the powder or granular cleansing composition (product) is preferably 80 µm or larger and 500 µm or smaller, more preferably 90 µm or larger and 400 µm or smaller, further more preferably 100 µm or larger and 300 µm or smaller. The carbonate is preferably prepared into a granulation product from the viewpoint of improving feeling in use and, particularly, reducing roughness in texture. In this case, the median diameter preferably falls within the range described above after granulation of the carbonate and before blending. The median diameter of the granulation product is in the suitable range described above as well as even more preferably 110 µm or larger and 300 µm or smaller, even more preferably 120 µm or larger and 300 µm or smaller, even more preferably 120 µm or larger and 200 µm or smaller, from the viewpoint of more improving preservation stability. In the case of using the starting material carbonate as it is, a preferred range of the median diameter of the carbonate in the powder or granular cleansing composition (product) is as described about the median diameter of the carbonate as starting material particles mentioned above.

The median diameter of the organic acid in the powder or granular cleansing composition (product) is preferably 70 µm or larger, more preferably 80 µm or larger, further more preferably 90 µm or larger, even more preferably 100 µm or larger, even more preferably 110 µm or larger, even more preferably 120 µm or larger, from the viewpoint of preservation stability. The median diameter is preferably 500 µm or smaller, more preferably 400 µm or smaller, further more preferably 300 µm or smaller, even more preferably 200 µm or smaller, from the viewpoint of productivity. The median diameter of a granulation product of the organic acid in the powder or granular cleansing composition (product) is preferably 70 µm or larger and 500 µm or smaller, more preferably 80 µm or larger and 500 µm or smaller, further more preferably 90 µm or larger and 400 µm or smaller, even more preferably 100 µm or larger and 300 µm or smaller. The organic acid is preferably prepared into a granulation product from the viewpoint of improving feeling in use and, particularly, reducing roughness in texture. In this case, the median diameter preferably falls within the range described above after granulation of the organic acid and before blending. The median diameter of the granulation product is in the suitable range described above as well as even more preferably 110 µm or larger and 300 µm or smaller, even more preferably 120 µm or larger and 300 µm or smaller, even more preferably 120 µm or larger and 200 µm or smaller, from the viewpoint of more improving preservation stability. In the case of using the starting material organic acid as it is, a preferred range of the median diameter of the organic acid in the powder or granular cleansing composition (product) is as described about the median diameter of the organic acid as starting material particles mentioned above.

In this context, the median diameter (D50) means a particle size at which a cumulative volume frequency calculated from volume fractions is 50% when calculated from smaller toward larger particle sizes. Specifically, the median diameter can be measured by a method described in Examples.

The starting material particles may be granulated after particles having a particle size of preferably 500 µm or larger, more preferably 400 µm or larger, further more preferably 300 µm or larger, are removed therefrom in advance using a sieve, from the viewpoint of attaining more favorable texture.

An additional starting material component used other than the carbonate and the organic acid has a median diameter equal to or smaller than the median diameters of the carbonate and the organic acid, and the median diameter is usually 170 µm or smaller, for example, 5 µm or larger and 150 µm or smaller.

Any of a fluidized-bed granulation method, a stirring granulation method, a tumbling granulation method, and an extrusion granulation method can be used as a granulation method. Among those described above, a fluidized-bed granulation method is preferred from the viewpoint of improving the solubility of the powder or granular cleansing composition in water.

Specifically, granulation can, for example, be carried out by charging starting material particles into a fluidized-bed granulator, and adding a binder solution of a binder dissolved in water, ethanol, or a mixed liquid thereof to a uniformly mixed powder and granular material.

The content (solid content) of the binder in the binder solution is preferably 0.1% by mass or more, more preferably 0.3% by mass or more, further more preferably 0.5% by mass or more, and preferably 10% by mass or less, more preferably 8% by mass or less, further more preferably 5% by mass or less, from the viewpoint of productivity. The content of the binder in the binder solution is preferably from 0.1 to 10% by mass, more preferably from 0.3 to 8% by mass, further more preferably from 0.5 to 5% by mass.

As mentioned above, the carbonate and the organic acid are preferably not contained at the same time in the same granulated particle from the viewpoint of improving preservation stability.

The granulated particles can be mixed with ungranulated particles by a known means to produce a cleansing composition. In the case of separately granulating the carbonate and the organic acid, the respective granulated particles (granulated particles containing the carbonate and granulated particles containing the organic acid) can be similarly mixed by use of a known means to produce a cleansing composition.

### <Method for using powder or granular cleansing composition>

The powder or granular cleansing composition according to the second embodiment of the present invention can be suitably used for washing, for example, the face, hair, or the body.

The powder or granular cleansing composition according to the second embodiment of the present invention is a foamable cleansing composition which starts to foam spontaneously by the addition of water. Hence, the composition can be conveniently utilized because the composition makes quick lather with the surfactant-containing composition and eliminates the need of a special procedure of making lather, which requires time and labor.

The method for using the powder or granular cleansing composition according to the second embodiment of the present invention is performed, for example, by putting the cleansing composition on the palm, adding water to the cleansing composition, thoroughly blending the water into the cleansing composition on the palm, then lightly making lather, and applying the foam to a recipient such as the face, hair, or the whole body for washing.

The amount of the water to be added to the powder or granular cleansing composition is preferably 1/30 or more, more preferably 1/25 or more, further more preferably 1/20 or more, and preferably 1/0.5 or less, more preferably 1/1 or less, further more preferably 1/2 or less, in terms of the mass ratio between the powder or granular cleansing composition and the water to be added (powder or granular cleansing composition/water) from the viewpoint of improving the solubility of the powder or granular cleansing composition and also improving foamability.

The powder or granular cleansing composition according to the second embodiment of the present invention is applied to the skin within a time of preferably 5 minutes, more preferably 3 minutes, further more preferably 2 minutes, even more preferably 1.5 minutes, after addition of water from the viewpoint of the retention of foam.

The temperature of the water to be added to the powder or granular cleansing composition is preferably 15°C or higher and 50°C or lower, more preferably 15°C or higher and 45°C or lower, further more preferably 20°C or higher and 45°C or lower, though it is not particularly limited.

Hereinafter, the present invention further discloses the following <1> to <27> in relation to the first embodiment mentioned above.
<1> A powder or granular carbonic acid foaming composition containing
   a carbonate and an organic acid, wherein
   particles having a particle size of 100 µm or larger are contained at 30% by mass or more, and
   a content of particles having a particle size of 100 µm or larger and having a hardness of 5 gf or higher is 0.10% by number or less.
<2> The powder or granular carbonic acid foaming composition according to <1>, wherein the particles having a hardness of 5 gf or higher are of 45% by number or less in the particles having a particle size of 100 µm or larger.
<3> The powder or granular carbonic acid foaming composition according to <1> or <2>, further containing a moisture absorbent.
<4> The powder or granular carbonic acid foaming composition according to any one of <1> to <3>, wherein the organic acid contains one or more members selected from the group consisting of citric acid, succinic acid, tartaric acid, and ascorbic acid.
<5> The powder or granular carbonic acid foaming composition according to any one of <1> to <4>, wherein the carbonate contains one or more members selected from the group consisting of sodium carbonate and sodium bicarbonate.
<6> The powder or granular carbonic acid foaming composition according to any one of <3> to <5>, wherein the moisture absorbent is magnesium oxide.
<7> The powder or granular carbonic acid foaming composition according to any one of <1> to <6>, wherein at least a part of the particles having a particle size of 100 µm or larger is granulated particles.
<8> The powder or granular carbonic acid foaming composition according to any one of <1> to <7>, wherein the particles having a particle size of 100 µm or larger are contained at 30% by mass or more and 100% by mass or less in all particles constituting the powder or granular carbonic acid foaming composition.
<9> The powder or granular carbonic acid foaming composition according to any one of <1> to <8>, wherein the particles having a particle size of 100 µm or larger are contained at 0.015% by number or more and 1% by number or less in all particles constituting the powder or granular carbonic acid foaming composition.
<10> The powder or granular carbonic acid foaming composition according to any one of <1> to <9>, wherein a content of a fine powder having a particle size of 10 µm or smaller is 0.001% by mass or more and 3.5% by mass or less in all particles constituting the powder or granular carbonic acid foaming composition.
<11> The powder or granular carbonic acid foaming composition according to any one of <7> to <10>, wherein a content of the granulated particles in the particles having a particle size of 100 µm or larger is more than 55% by number.
<12> The powder or granular carbonic acid foaming composition according to any one of <1> to <11>, wherein an average hardness value of the particles having a particle size of 100 µm or larger is 0.3 gf or higher and lower than 5 gf.
<13> The powder or granular carbonic acid foaming composition according to any one of <1> to <12>, wherein the particles having a particle size of 100 µm or larger are of 35% by mass or more and 100% by mass or less and of 0.017% by number or more and 0.7% by number or less.
<14> The powder or granular carbonic acid foaming composition according to any one of <1> to <13>, wherein the content of the particles having a particle size of 100 µm or larger and having a hardness of 5 gf or higher is 0.07% by number or less in all particles constituting the powder or granular carbonic acid foaming composition, and the particles having a hardness of 5 gf or higher are of 40% by number or less in the particles having a particle size of 100 µm or larger.
<15> The powder or granular carbonic acid foaming composition according to any one of <1> to <14>, wherein a content of the carbonate in the powder or granular carbonic acid foaming composition is 15% by mass or more and 65% by mass or less.
<16> The powder or granular carbonic acid foaming composition according to any one of <1> to <15>, wherein a content of the organic acid in the powder or granular carbonic acid foaming composition is 5% by mass or more and 60% by mass or less.
<17> The powder or granular carbonic acid foaming composition according to any one of <1> to <16>, wherein a mass ratio of the organic acid to the carbonate [organic acid/carbonate] is 0.05 or more and 10 or less.
<18> The powder or granular carbonic acid foaming composition according to any one of <1> to <17>, wherein a total amount of the carbonate and the organic acid in the powder or granular carbonic acid foaming composition is from 50 to 90% by mass.
<19> The powder or granular carbonic acid foaming composition according to any one of <3> to <18>, wherein a content of the moisture absorbent in the powder or granular carbonic acid foaming composition is from 0.01 to 15% by mass.
<20> The powder or granular carbonic acid foaming composition according to any one of <1> to <19>, wherein the composition contains particles (A) containing the carbonate and particles (B) containing the organic acid, wherein
   the particles (A) are particles containing no organic acid, and the particles (B) are particles containing no carbonate.
<21> The powder or granular carbonic acid foaming composition according to any one of <1> to <19>, wherein the particles (A) contain the excipient.
<22> The powder or granular carbonic acid foaming composition according to any one of <1> to <19>, wherein the particles (B) contain the excipient.
<23> The powder or granular carbonic acid foaming composition according to any one of <1> to <19>, wherein both the particles (A) and the particles (B) contain the excipient.
<24> A method for producing the powder or granular carbonic acid foaming composition according to any one of <1> to <23>, containing a step of
   granulating at least one of the carbonate and the organic acid.
<25> A method for using the powder or granular carbonic acid foaming composition according to any one of <1> to <23>, containing bringing the powder or granular carbonic acid foaming composition into contact with a surfactant-containing composition to form the surfactant-containing composition into a dosage form of foam.
<26> The method for using the powder or granular carbonic acid foaming composition according to <25>, wherein the surfactant-containing composition is a hair cosmetic.
<27> A hair cosmetic kit containing the powder or granular carbonic acid foaming composition according to any one of <1> to <23> and a surfactant-containing composition.

The present invention further discloses the following <31> to <57> in relation to the second embodiment mentioned above.
<31> A powder or granular cleansing composition containing
   a carbonate, an organic acid, and a surfactant, wherein
   particles having a particle size of 100 µm or larger are contained at 30% by mass or more, and
   a content of particles having a particle size of 100 µm or larger and having a hardness of 5 gf or higher is 0.10% by number or less.
<32> The powder or granular cleansing composition according to <31>, wherein the particles having a hardness of 5 gf or higher are of 45% by number or less in the particles having a particle size of 100 µm or larger.
<33> The powder or granular cleansing composition according to <31> or <32>, further containing a moisture absorbent.
<34> The powder or granular cleansing composition according to any one of <31> to <33>, wherein the organic acid contains one or more members selected from the group consisting of citric acid, succinic acid, tartaric acid, and ascorbic acid.
<35> The powder or granular cleansing composition according to any one of <31> to <34>, wherein the carbonate contains one or more members selected from the group consisting of sodium carbonate and sodium bicarbonate.
<36> The powder or granular cleansing composition according to any one of <33> to <35>, wherein the moisture absorbent is magnesium oxide.
<37> The powder or granular cleansing composition according to any one of <31> to <36>, wherein at least a part of the particles having a particle size of 100 µm or larger is granulated particles.
<38> The powder or granular cleansing composition according to <37>, wherein the granulated particles are granulated particles obtained by granulating any one of the organic acid and the carbonate, and an additional component, other than the organic acid and the carbonate, constituting the cleansing composition.
<39> The powder or granular cleansing composition according to any one of <31> to <38>, wherein the particles having a particle size of 100 µm or larger are contained at 30% by mass or more and 100% by mass or less in all particles constituting the powder or granular cleansing composition.
<40> The powder or granular cleansing composition according to any one of <31> to <39>, wherein the particles having a particle size of 100 µm or larger are contained at 0.015% by number or more and 1% by number or less in all particles constituting the powder or granular cleansing composition.
<41> The powder or granular cleansing composition according to any one of <31> to <40>, wherein a content of a fine powder having a particle size of 10 µm or smaller is 0.001% by mass or more and 3.5% by mass or less in all particles constituting the powder or granular cleansing composition.
<42> The powder or granular cleansing composition according to any one of <31> to <41>, wherein an average hardness value of the particles having a particle size of 100 µm or larger is 0.3 gf or higher and lower than 5 gf.
<43> The powder or granular cleansing composition according to any one of <37> to <42>, wherein a content of the granulated particles among the particles having a particle size of 100 µm or larger is more than 55% by number.
<44> The powder or granular cleansing composition according to any one of <31> to <43>, wherein the particles having a particle size of 100 µm or larger are of 35% by mass or more and 100% by mass or less and of 0.017% by number or more and 0.7% by number or less.
<45> The powder or granular cleansing composition according to any one of <31> to <44>, wherein the content of the particles having a particle size of 100 µm or larger and having a hardness of 5 gf or higher is 0.07% by number or less in all particles constituting the powder or granular cleansing composition, and the particles having a hardness of 5 gf or higher are of 40% by number or less in the particles having a particle size of 100 µm or larger.
<46> The powder or granular cleansing composition according to any one of <31> to <45>, wherein a content of the carbonate is 10% by mass or more and 60% by mass or less.
<47> The powder or granular cleansing composition according to any one of <31> to <46>, wherein a content of the organic acid is 5% by mass or more and 60% by mass or less.
<48> The powder or granular cleansing composition according to any one of <31> to <47>, wherein a content of the surfactant is 10% by mass or more and 50% by mass or less.
<49> The powder or granular cleansing composition according to any one of <31> to <48>, wherein a mass ratio of a total amount of the carbonate and the organic acid to the surfactant [(carbonate + organic acid)/surfactant] is 0.1 or more and 20 or less.
<50> The powder or granular cleansing composition according to any one of <31> to <49>, wherein a mass ratio of the organic acid to the carbonate [organic acid/carbonate] is 0.05 or more and 10 or less.
<51> The powder or granular cleansing composition according to any one of <31> to <50>, wherein a total amount of the carbonate and the organic acid is 35% by mass or more and 70% by mass or less.
<52> The powder or granular cleansing composition according to any one of <33> to <51>, wherein a content of the moisture absorbent is 0.01% by mass or more and 10% by mass or less.
<53> The powder or granular cleansing composition according to any one of <31> to <52>, wherein the composition contains particles (A) containing the carbonate and particles (B) containing the organic acid, wherein
   the particles (A) are particles containing no organic acid, and the particles (B) are particles containing no carbonate.
<54> The powder or granular cleansing composition according to <53>, wherein the particles (A) contain the surfactant.
<55> The powder or granular cleansing composition according to <53>, wherein the particles (B) contain the surfactant.
<56> The powder or granular cleansing composition according to <53>, wherein both the particles (A) and the particles (B) contain the surfactant.
<57> A method for producing the powder or granular cleansing composition according to any one of <31> to <56>, containing a step of
   granulating at least one of a carbonate and an organic acid.

### Examples

Hereinafter, the present invention will be described with reference to Examples. However, the present invention is not limited by the scope of Examples. Measurement in the present Examples was performed by the following methods.

### (1) Method for measuring content (proportion by number) of particles having particle size of 100 µm or larger

A particle size distribution was measured with CAMSIZER XT (particle size measurement apparatus, manufactured by Retsch GmbH) using 3 g of a powder or granular carbonic acid foaming composition or a powder or granular cleansing composition. Particle sizes of from 1 to 3 000 µm in the obtained distribution were evenly divided into 100 intervals as logarithms. Particle sizes in a range equal to or larger than the interval involving 100 µm were counted as 100 µm or larger. The total number of 100 µm or larger particles was calculated, and the content ratio (proportion by number) of the particles having a particle size of 100 µm or larger in all the particles was calculated.

### (2) Method for measuring content (proportion by number) of particles having hardness of 5 gf or higher in particles having particle size of 100 µm or larger

A powder or granular carbonic acid foaming composition or a powder or granular cleansing composition was sieved through a sieve with an opening of 100 µm, and particles on the sieve were collected with a very small spatula. Next, the particles collected with a spatula were spread over a press plate of a micro compression tester (manufactured by Shimadzu Corp., MCTW500). The hardnesses of arbitrary 20 particles were measured, and the content (proportion by number) of the particles having a hardness of 5 gf or higher in the particles having a particle size of 100 µm or larger was calculated. This operation was repeated three times, and an average value was calculated.

### (3) Method for calculating content of particles having particle size of 100 µm or larger and hardness of 5 gf or higher

The content of the particles having a particle size of 100 µm or larger and having a hardness of 5 gf or higher in all the particles was calculated by multiplying the numeric value obtained by the measurement of the section (1) by the numeric value obtained by the measurement of the section (2).

### (4) Method for measuring content (proportion by number) of granulated particles in particles having particle size of 100 µm or larger

A powder or granular cleansing composition was sieved through a sieve with an opening of 100 µm, and particles on the sieve were collected with a very small spatula. The collected particles were spread over a 5 mm × 5 mm carbon tape and observed under Hitachi Tabletop Microscope Miniscope TM3030 (manufactured by Hitachi High-Tech Corp.). Arbitrary 20 grains were observed, and the content (proportion by number) of aggregates as the granulated particles was calculated.

### (5) Method for measuring content (proportion by mass) of particles having particle size of 100 µm or larger

3 g of a powder or granular carbonic acid foaming composition or a powder or granular cleansing composition was sieved through a sieve with an opening of 100 µm, and the content was calculated from the mass of particles remaining on the upper part.

### (6) Method for measuring content of fine powder

A particle size distribution was measured with CAMSIZER XT (particle size measurement apparatus, manufactured by Retsch GmbH) using 3 g of a powder or granular carbonic acid foaming composition or a powder or granular cleansing composition. Particle sizes of from 1 to 3 000 µm in the obtained distribution were evenly divided into 100 intervals as logarithms. Particle sizes in a range equal to or smaller than the interval involving 10 µm were counted as 10 µm or smaller. A volume frequency at 10 µm or smaller was calculated, and the content (proportion by mass) of particles having a particle size of 10 µm or smaller in all the particles was calculated.

### [Method for measuring median diameter]

Starting materials organic acid and carbonate, and their granulation products were each used in an amount of 3 g, and their median diameters were measured with CAMSIZER XT (particle size measurement apparatus, manufactured by Retsch GmbH).

### [Preparation of mixed surfactant]

300 g of sodium N-myristoyl-L-glutamate (Amisoft MS manufactured by Ajinomoto Co., Inc.) and 291.5 g of sodium N-lauroyl-L-glutamate (Amisoft LS manufactured by Ajinomoto Co., Inc.) were mixed at room temperature to give a mixed surfactant.

### <First embodiment>

### [Example 1-1]

A powder or granular carbonic acid foaming composition was obtained in accordance with the formulation shown in Table 1.

A fluidized-bed granulator (FD-MP-01E manufactured by Powrex Corp.) was charged with 158.6 g of citric acid (Citric Anhydride 60 manufactured by Iwata Chemical Co., Ltd.), 39.5 g of magnesium oxide (manufactured by Kyowa Chemical Industry Co., Ltd.), 84.7 g of talc (SW-K4 manufactured by Asada Milling Co., Ltd.), and 16.3 g of cationized hydroxyethylcellulose (SOFTCAT Polymer SL-30 manufactured by The Dow Chemical Company), which were then granulated under conditions involving an air flow of 0.2 m³/min and a suction temperature of 80°C while 90 g (solid content: 0.9 g) of an aqueous solution of 1.0% carboxymethylcellulose (manufactured by Nippon Paper Industries Co., Ltd., SUNROSE; hereinafter, also referred to as CMC) was added at a rate of 4 g/min, to give a granulation product having a median diameter of 175 µm. The obtained granulation product and 264.3 g of sodium bicarbonate (hereinafter, also referred to as baking soda; manufactured by AGC Inc.) were added to a bag and manually mixed until uniform in a clear vinyl bag to prepare a powder or granular carbonic acid foaming composition.

The obtained powder or granular carbonic acid foaming composition was evaluated for each item in accordance with a method given below. The results are shown in Table 1. Table 2 shows the particle sizes of citric acid and sodium bicarbonate (baking soda) used as starting materials and their respective particle sizes in the powder or granular carbonic acid foaming composition (product) obtained by production.

### [Examples 1-2 and 1-3]

Each powder or granular carbonic acid foaming composition was obtained by the same method as in Example 1-1 except that the formulation was as shown in Table 1.

The obtained powder or granular carbonic acid foaming composition was evaluated for each item in accordance with a method given below. The results are shown in Table 1. Table 2 shows the particle sizes of citric acid and baking soda used as starting materials and their respective particle sizes in the powder or granular carbonic acid foaming composition (product) obtained by production.

### [Example 1-4]

Sodium bicarbonate was granulated in accordance with the formulation shown in Table 1. A fluidized-bed granulator (FD-MP-01E manufactured by Powrex Corp.) was charged with 195.3 g of sodium bicarbonate (manufactured by AGC Inc.), 29.2 g of magnesium oxide (manufactured by Kyowa Chemical Industry Co., Ltd.), 62.6 g of talc (SW-K4 manufactured by Asada Milling Co., Ltd.), and 12.0 g of cationized hydroxyethylcellulose (SOFTCAT Polymer SL-30 manufactured by The Dow Chemical Company), which were then granulated under the same conditions as in Example 1-1 while 66 g (solid content: 0.7 g) of an aqueous solution of 1.0% CMC was added at a rate of 4 g/min, to give a granulation product having a median diameter of 145 µm. The obtained granulation product and 117.2 g of citric acid (Citric Anhydride 60 manufactured by Iwata Chemical Co., Ltd.) were manually mixed until uniform in a clear vinyl bag to prepare a powder or granular carbonic acid foaming composition.

The obtained powder or granular carbonic acid foaming composition was evaluated for each item in accordance with a method given below. The results are shown in Table 1. Table 2 shows the particle sizes of citric acid and sodium bicarbonate used as starting materials and their respective particle sizes in the powder or granular carbonic acid foaming composition (product) obtained by production.

### [Example 1-5]

A powder or granular carbonic acid foaming composition was obtained by the same method as in Example 1-1 except that citric acid (Fine Citric Acid Powder manufactured by Iwata Chemical Co., Ltd.) was used.

The obtained powder or granular carbonic acid foaming composition was evaluated for each item in accordance with a method given below. The results are shown in Table 1. Table 2 shows the particle sizes of citric acid and baking soda used as the starting materials and their respective particle sizes in the powder or granular carbonic acid foaming composition (product) obtained by production.

### [Example 1-6]

A carbonic acid foaming composition was obtained by the same method as in Example 1-1 except that 142.9 g of citric acid (Citric Anhydride 60 manufactured by Iwata Chemical Co., Ltd.), 37.6 g of magnesium oxide (manufactured by Kyowa Chemical Industry Co., Ltd.), 75.8 g of talc (SW-K4 manufactured by Asada Milling Co., Ltd.), 15.6 g of cationized hydroxyethylcellulose (SOFTCAT Polymer SL-30 manufactured by The Dow Chemical Company), and 26.9 g of sodium cocoyl isethionate (JODAPONLACI manufactured by BASF ED) were used.

The obtained carbonic acid foaming composition was evaluated for each item in accordance with a method given below. The results are shown in Table 1. Table 2 shows the particle sizes of citric acid and baking soda used as starting materials and their respective particle sizes in the powder or granular carbonic acid foaming composition (product) obtained by production.

### [Comparative Examples 1-1 to 1-3]

Each powder or granular carbonic acid foaming composition was obtained in accordance with the formulation shown in Table 3.

In Comparative Examples 1-1 to 1-3, all components were added to a bag without being granulated, and manually mixed until uniform in a clear vinyl bag to give a powder or granular carbonic acid foaming composition.

The obtained powder or granular carbonic acid foaming composition was evaluated for each item in accordance with a method given below. The results are shown in Table 3. Table 4 shows the particle sizes of citric acid and baking soda used as the starting materials and their respective particle sizes in the powder or granular carbonic acid foaming composition (product) obtained by production.

### [Comparative Example 1-4]

A powder or granular carbonic acid foaming composition was obtained in accordance with the formulation shown in Table 3.

All components were added to a bag without being granulated, and manually mixed until uniform in a clear vinyl bag. 1 g of the obtained mixture was placed in a mold of ϕ4 mm and pressed by hand at 10 MPa to give a compression-molded product of ϕ4 mm.

The obtained compression-molded product was pulverized in a mortal and sieved through a 250 µm sieve, and a pass-through portion was separated to give a powder or granular carbonic acid foaming composition having a median diameter of 176 µm.

### [Foamability (without addition of water)]

1 g of each powder or granular carbonic acid foaming composition was put on the palm, and 6 g of a commercially available shampoo liquid (Kao Corp., Essential The Beauty) was added to the powder or granular carbonic acid foaming composition. The shampoo liquid was thoroughly blended into the powder or granular carbonic acid foaming composition on the palm and allowed to foam spontaneously. Then (after approximately 10 seconds from the addition of the shampoo liquid), these were mixed by fingers of the other hand to make lather. Then, foamability in making lather within 3 minutes from the addition of the shampoo liquid in a state in which the operation of making lather was continued was evaluated by three specialist panels in accordance with the following criteria and determined by the discussion among the specialist panels.
1: A palmful of foam was made in shorter than 30 seconds
2: A palmful of foam was made in 30 seconds or longer and shorter than 1 minute
3: A palmful of foam was made in 1 minute or longer and shorter than 2 minutes
4: A palmful of foam was made in 2 minutes or longer and shorter than 3 minutes
5: Sufficient foaming took 3 minutes or longer

### [Foamability (with addition of water)]

1 g of each powder or granular carbonic acid foaming composition was put on the palm, and 5 g of water of 42°C was added to the powder or granular carbonic acid foaming composition. The water was thoroughly blended into the powder or granular carbonic acid foaming composition on the palm and allowed to foam spontaneously. Then (after a lapse of approximately 10 seconds from the addition of the water), 6 g of a commercially available shampoo liquid (Kao Corp., Essential The Beauty) was added thereto, and the shampoo liquid was thoroughly blended into the powder or granular carbonic acid foaming composition on the palm and allowed to foam spontaneously. Then, these were mixed by fingers of the other hand to make lather. Then, foamability in making lather within 3 minutes from the addition of the water in a state in which the operation of making lather was continued was evaluated by three specialist panels in accordance with the following criteria and determined by the discussion among the specialist panels.
1: A palmful of foam was made in shorter than 30 seconds
2: A palmful of foam was made in 30 seconds or longer and shorter than 1 minute
3: A palmful of foam was made in 1 minute or longer and shorter than 2 minutes
4: A palmful of foam was made in 2 minutes or longer and shorter than 3 minutes
5: Making a palmful of foam took 3 minutes or longer

### [Preservation stability]

3.9 g of each powder or granular carbonic acid foaming composition was enclosed in an 80 mm × 50 mm × 18 mm aluminum packaging material at 50°C under 50% RH and stored at 50°C for 4 weeks. The amount of the aluminum packaging material swelling was measured before and after the storage. Specifically, a water bath filled with water of 25°C was placed on a scale, and its weight was measured. Next, the aluminum packaging material in which the powder or granular carbonic acid foaming composition was enclosed was completely submerged in the water bath, and its mass was measured. The difference in mass between before and after the aluminum packaging material was submerged in water was converted to a volume with the density of water defined as 1.0 g/ml in accordance with the principle of Archimedes, and this volume was regarded as the volume of the aluminum packaging material before storage. Then, the same measurement as above was performed after storage for 4 weeks to determine the volume of the aluminum packaging material after the storage. The amount of the aluminum packaging material swelling after the storage was determined from the difference in volume of the aluminum packaging material between before and after the storage. A smaller amount of the packaging material swelling indicates better preservation stability.

### [Feeling in use (texture/without addition of water)]

1 g of each powder or granular carbonic acid foaming composition was put on the palm, and 6 g of a commercially available shampoo liquid (Kao Corp., Essential The Beauty) was added to the powder or granular carbonic acid foaming composition. The shampoo liquid was thoroughly blended into the powder or granular carbonic acid foaming composition on the palm and allowed to foam spontaneously. Then, these were mixed by fingers of the other hand to make lather.

Then, texture in making lather within 3 minutes from the addition of the shampoo liquid to the powder or granular carbonic acid foaming composition in a state in which the operation of making lather was continued was evaluated by three specialist panels in accordance with the following criteria and determined by the discussion among the specialist panels.
1: No roughness was felt from the beginning
2: Roughness disappeared at the initial stage of making lather, though felt first
3: Roughness disappeared during making lather, though felt first
4: Roughness disappeared after making lather, though felt
5: Roughness was felt and did not disappear even after making lather
(The phrase "initial stage of making lather" refers to within 1 minute from the addition of the shampoo liquid to the powder or granular carbonic acid foaming composition; the phrase "during making lather" refers to longer than 1 minute and within 2 minutes from the addition; and the phrase "after making lather" refers to longer than 2 minutes and within 3 minutes from the addition.)

### [Feeling in use (texture/with addition of water)]

1 g of each carbonic acid foaming composition was put on the palm, and 5 g of water of 42°C was added to the carbonic acid foaming composition. The water was thoroughly blended into the carbonic acid foaming composition on the palm and allowed to foam spontaneously. Then (after a lapse of approximately 10 seconds from the addition of the water), 6 g of a commercially available shampoo liquid (Kao Corp., Essential The Beauty) was added thereto, and the shampoo liquid was thoroughly blended into the carbonic acid foaming composition on the palm and allowed to foam spontaneously. Then, these were mixed by fingers of the other hand to make lather. Then, texture in making lather within 3 minutes from the addition of the water in a state in which the operation of making lather was continued was evaluated by three specialist panels in accordance with the following criteria and determined by the discussion among the specialist panels.
1: No roughness was felt from the beginning
2: Roughness disappeared at the initial stage of making lather, though felt first
3: Roughness disappeared during making lather, though felt first
4: Roughness disappeared after making lather, though felt
5: Roughness was felt and did not disappear even after making lather
(The phrase "initial stage of making lather" refers to within 1 minute from the addition of the water to the carbonic acid foaming composition; the phrase "during making lather" refers to longer than 1 minute and within 2 minutes from the addition; and the phrase "after making lather" refers to longer than 2 minutes and within 3 minutes from the addition.)

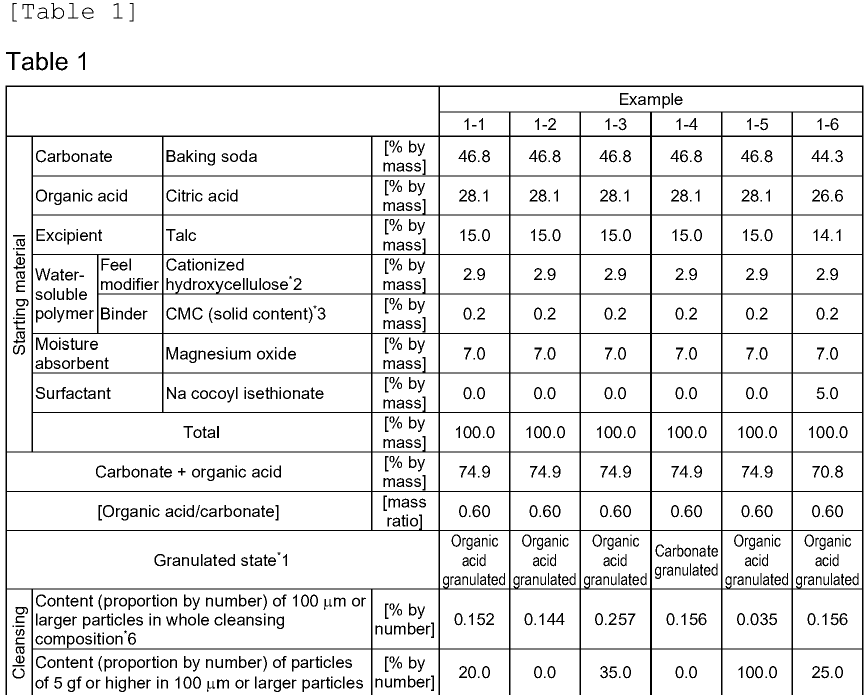

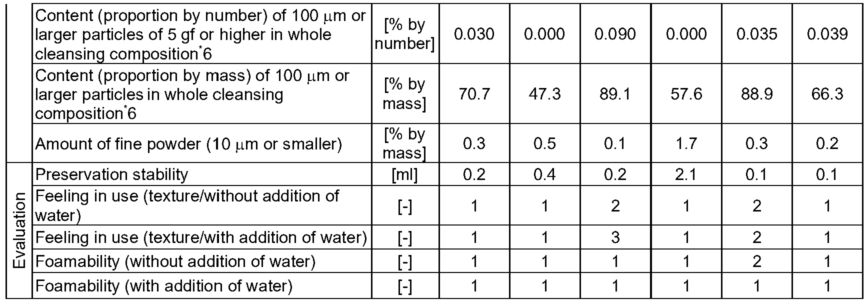

### [Table 2]

**Table 2**

| | | | Example | | | | | |
|---|---|---|---|---|---|---|---|---|
| | | | 1-1 | 1-2 | 1-3 | 1-4 | 1-5 | 1-6 |
| Granulated state^{*}1 | | | Organic acid granulated | Organic acid granulated | Organic acid granulated | Carbonate granulated | Organic acid granulated | Organic acid granulated |
| Baking soda particle size (median diameter) | Starting material | [µm] | 100 | 57 | 160 | 57 | 100 | 100 |
| | Granulation product | [µm] | - | - | - | 145 | - | - |
| Citric acid particle size (median diameter) | Starting material | [µm] | 38 | 38 | 38 | 38 | 326 | 38 |
| | Granulation product | [µm] | 175 | 175 | 175 | - | 384 | 160 |

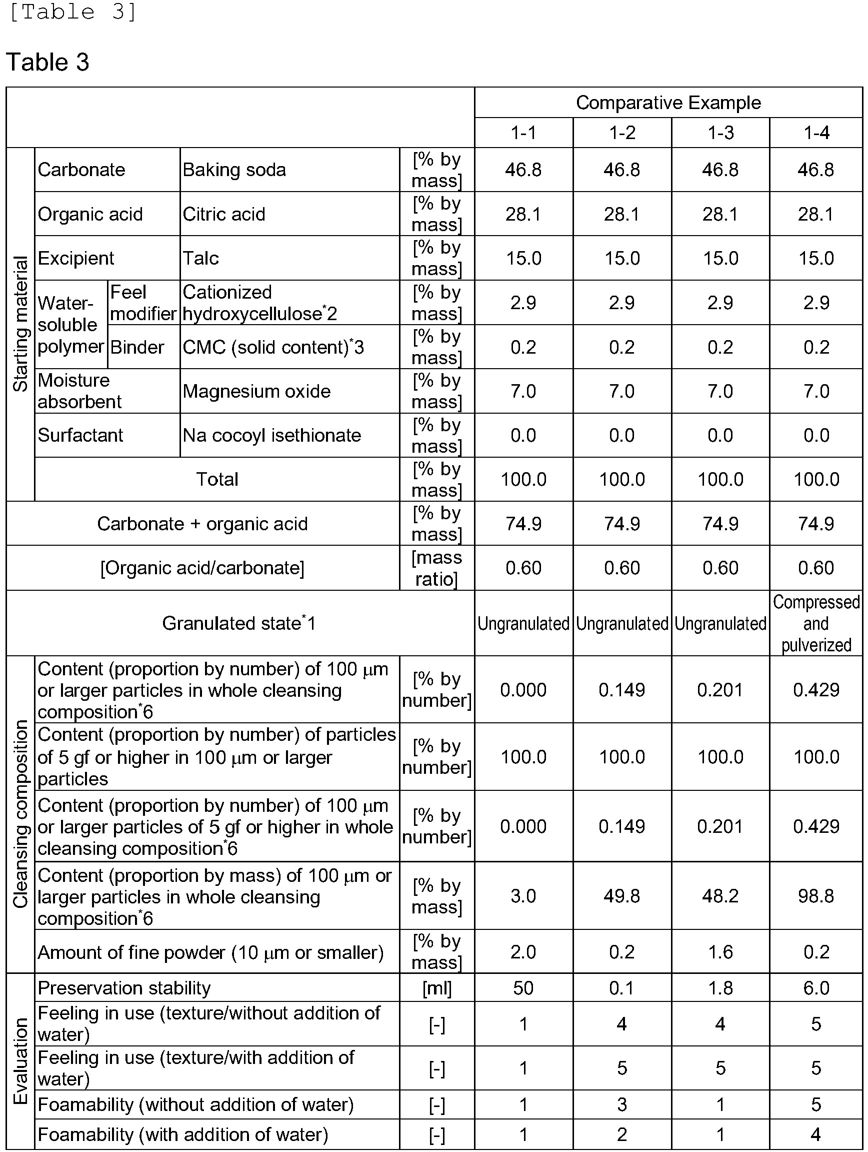

### [Table 4]

**Table 4**

| | | | Comparative Example | | | |
|---|---|---|---|---|---|---|
| | | | 1-1 | 1-2 | 1-3 | 1-4 |
| Granulated state^{*}1 | | | Ungranulated | Ungranulated | Ungranulated | Compressed and pulverized |
| Baking soda particle size (median diameter) | Starting material | [µm] | 57 | 100 | 176 | 57 |
| | Granulation product | [µm] | - | - | - | 176 |
| Citric acid particle size (median diameter) | Starting material | [µm] | 38 | 720 | 38 | 38 |
| | Granulation product | [µm] | - | - | - | 176 |

*1 In "Granulated state" in Tables 1 to 4, "Organic acid granulated" means that all the components (organic acid, excipient, water-soluble polymer, and moisture absorbent) other than the carbonate were granulated. In the case of "Organic acid granulated", the carbonate (baking soda) was used as ungranulated starting material particles.

"Carbonate granulated" means that the carbonate and all the components (excipient, water-soluble polymer, and moisture absorbent) other than the organic acid were granulated. In the case of "Carbonate granulated", the organic acid (citric acid) was used as ungranulated starting material particles.

"Ungranulated" refers to a state in which all the powder components were used without being granulated.
*2 SOFTCAT Polymer SL-30 manufactured by The Dow Chemical Company
*3 SUNROSE manufactured by Nippon Paper Industries Co., Ltd.
*6 In Tables 1 and 3, "in whole cleansing composition" means "in all particles constituting the powder or granular cleansing composition".

It is evident from Tables 1 and 3 that the powder or granular carbonic acid foaming compositions of the present Examples are excellent in preservation stability and excellent in texture.

All the shampoos to which the powder or granular carbonic acid foaming compositions of the present Examples were applied had favorable foamability, foam break, and long-lasting foam.

### <Second embodiment>

### [Example 2-1]

A powder or granular cleansing composition was obtained in accordance with the formulation shown in Table 5.

A fluidized-bed granulator (FD-MP-01E manufactured by Powrex Corp.) was charged with 67.4 g of citric acid (Citric Anhydride 60 manufactured by Iwata Chemical Co., Ltd.), 118.3 g of a mixed surfactant, 25.2 g of magnesium oxide (manufactured by Kyowa Chemical Industry Co., Ltd.), and 84.0 g of talc (SW-K4 manufactured by Asada Milling Co., Ltd.), which were then granulated under conditions involving an air flow of 0.3 m³/min and a suction temperature of 80°C while 314 g (solid content: 5.0 g) of an aqueous solution of 1.6% carrageenan was added at a rate of 5 g/min, to give a granulation product having a median diameter of 172 µm. The obtained granulation product and 203 g of sodium bicarbonate (hereinafter, also referred to as baking soda; manufactured by AGC Inc.) were added to a bag and manually mixed until uniform in a clear vinyl bag to prepare a powder or granular cleansing composition.

The obtained cleansing composition was evaluated for each item in accordance with a method given below. The results are shown in Table 5. Table 6 shows the particle sizes of citric acid and sodium bicarbonate (baking soda) used as the starting materials and their respective particle sizes in the powder or granular cleansing composition (product) obtained by production.

### [Examples 2-2 to 2-3 and 2-5 to 2-7]

Each powder or granular cleansing composition was obtained by the same method as in Example 2-1 except that the formulation was as shown in Table 5.

The obtained powder or granular cleansing composition was evaluated for each item in accordance with a method given below. The results are shown in Table 5. Table 6 shows the particle sizes of citric acid and baking soda used as starting materials and their respective particle sizes in the powder or granular cleansing composition (product) obtained by production.

### [Example 2-4]

Sodium bicarbonate was granulated in accordance with the formulation shown in Table 5. A fluidized-bed granulator (FD-MP-01E manufactured by Powrex Corp.) was charged with 139.9 g of sodium bicarbonate (manufactured by AGC Inc.), 81.5 g of a mixed surfactant, 17.3 g of magnesium oxide (manufactured by Kyowa Chemical Industry Co., Ltd.), and 57.8 g of talc (SW-K4 manufactured by Asada Milling Co., Ltd.), which were then granulated under the same conditions as in Example 2-1 while 216 g (solid content: 3.5 g) of an aqueous solution of 1.6% carrageenan was added at a rate of 5 g/min, to give a granulation product having a median diameter of 127 µm. The obtained granulation product and 46.6 g of citric acid were manually mixed until uniform in a clear vinyl bag to prepare a powder or granular cleansing composition.

The obtained powder or granular cleansing composition was evaluated for each item in accordance with a method given below. The results are shown in Table 5. Table 6 shows the particle sizes of citric acid and baking soda used as starting materials and their respective particle sizes in the powder or granular cleansing composition (product) obtained by production.

### [Comparative Examples 2-1 to 2-3]

Each powder or granular cleansing composition was obtained in accordance with the formulation shown in Table 7.

In Comparative Examples 2-1 to 2-3, all components were added to a bag without being granulated, and manually mixed in the bag to give a powder or granular cleansing composition.

The obtained powder or granular cleansing composition was evaluated for each item in accordance with a method given below. The results are shown in Table 7. Table 8 shows the particle sizes of citric acid and baking soda used as the starting materials.

### [Preservation stability]

3.9 g of each cleansing composition was enclosed in an 80 mm × 50 mm × 18 mm aluminum packaging material at 50°C under 50% RH and stored at 50°C for 4 weeks. The amount of the aluminum packaging material swelling was measured before and after the storage. Specifically, a water bath filled with water of 25°C was placed on a scale, and its weight was measured. Next, the aluminum packaging material in which the cleansing composition was enclosed was completely submerged in the water bath, and its mass was measured. The difference in mass between before and after the aluminum packaging material was submerged in water was converted to a volume with the density of water defined as 1.0 g/ml in accordance with the principle of Archimedes, and this volume was regarded as the volume of the aluminum packaging material before storage. Then, the same measurement as above was performed after storage for 4 weeks to determine the volume of the aluminum packaging material after the storage. The amount of the aluminum packaging material swelling after the storage was determined from the difference in volume of the aluminum packaging material between before and after the storage. A smaller amount of the packaging material swelling indicates better preservation stability.

### [Feeling in use (texture)]

1 g of each powder or granular cleansing composition was put on the palm, and 10 g of water of 42°C was added dropwise to the cleansing composition. The water was thoroughly blended into the cleansing composition on the palm and allowed to foam spontaneously. Then (after a lapse of approximately 10 seconds from the dropwise addition of the water), both the palms were brought together to make lather. Then, texture in making lather within 3 minutes from the dropwise addition of the water in a state in which the operation of making lather was continued was evaluated by three specialist panels in accordance with the following criteria and determined by the discussion among the specialist panels.
1: No roughness was felt from the beginning
2: Roughness disappeared at the initial stage of making lather, though felt first
3: Roughness disappeared during making lather, though felt first
4: Roughness disappeared after making lather, though felt
5: Roughness was felt and did not disappear even after making lather
(The phrase "initial stage of making lather" refers to within 1 minute from the dropwise addition of the water to the cleansing composition; the phrase "during making lather" refers to longer than 1 minute and within 2 minutes from the dropwise addition; and the phrase "after making lather" refers to longer than 2 minutes and within 3 minutes from the dropwise addition.)

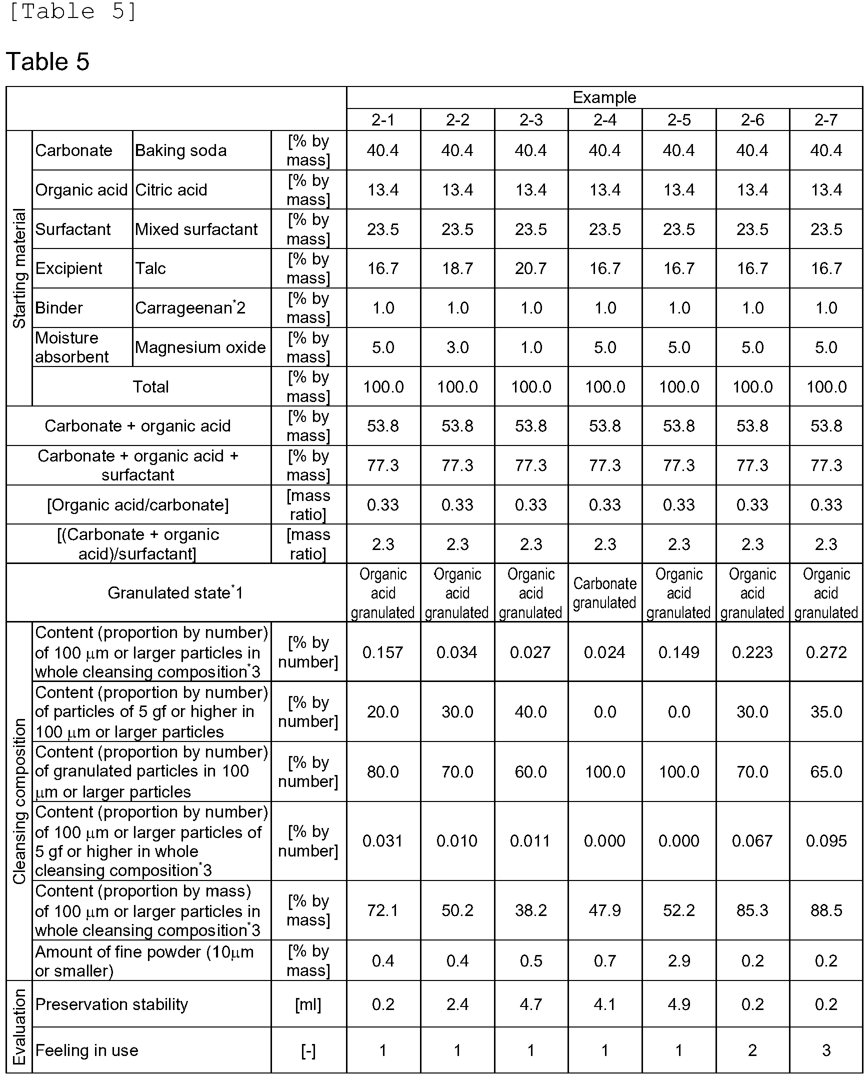

### [Table 6]

**Table 6**

| | | | Example | | | | | | |
|---|---|---|---|---|---|---|---|---|---|
| | | | 2-1 | 2-2 | 2-3 | 2-4 | 2-5 | 2-6 | 2-7 |
| Granulated state*1 | | | Organic acid granulated | Organic acid granulated | Organic acid granulated | Carbonate granulated | Organic acid granulated | Organic acid granulated | Organic acid granulated |
| Baking soda particle size^{*}4 | Starting material | [µm] | 100 | 100 | 100 | 41 | 41 | 147 | 162 |
| | Granulation product | [µm] | - | - | - | 127 | - | - | - |
| Citric acid particle size^{*}4 | Starting material | [µm] | 38 | 65 | 65 | 38 | 38 | 38 | 38 |
| | Granulation product | [µm] | 172 | 97 | 81 | - | 172 | 172 | 172 |

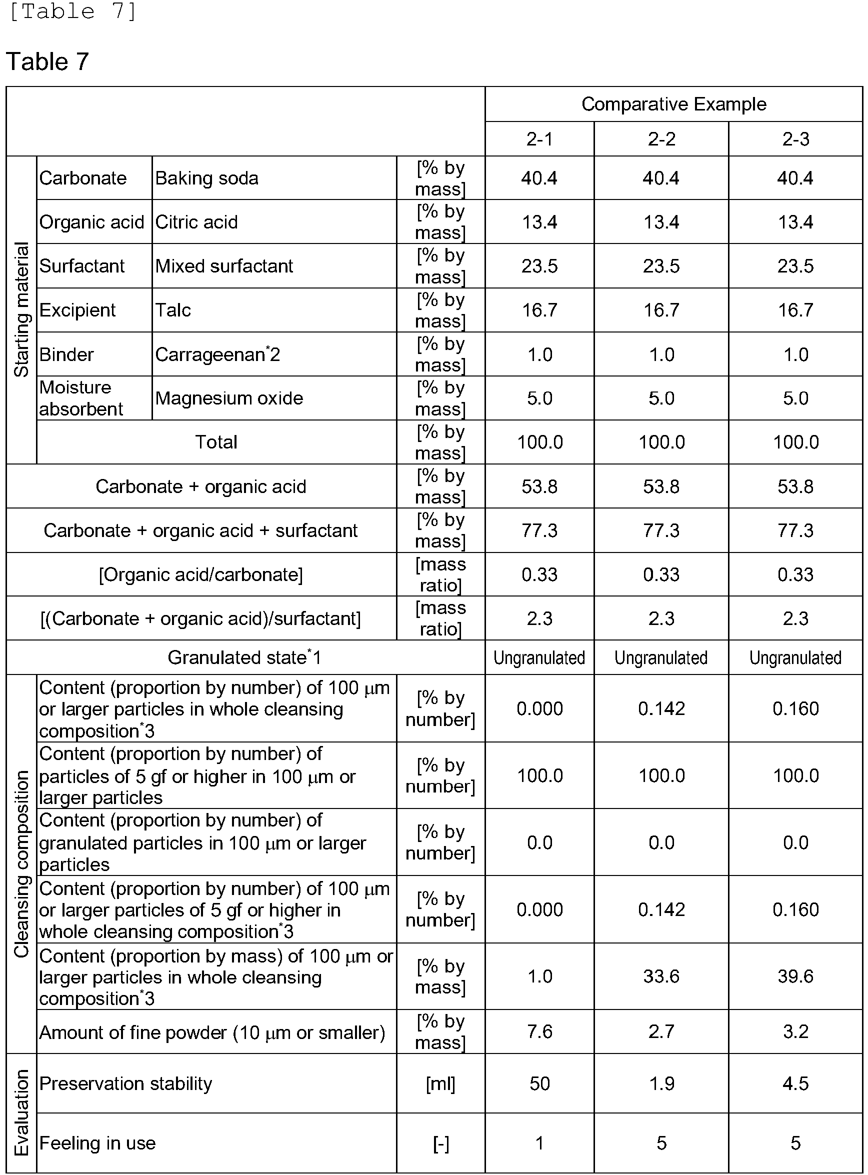

### [Table 8]

**Table 8**

| | | | Comparative Example | | |
|---|---|---|---|---|---|
| | | | 2-1 | 2-2 | 2-3 |
| Granulated state^{*}1 | | | Ungranulated | Ungranulated | Ungranulated |
| Baking soda particle size^{*}4 | Starting material | [µm] | 41 | 100 | 180 |
| | Granulation product | [µm] | - | - | - |
| Citric acid particle size^{*}4 | Starting material | [µm] | 38 | 720 | 38 |
| | Granulation product | [µm] | - | - | - |

*1 "Organic acid granulated" described in the section "Granulated state" in Tables 5 to 8, refers to a state in which the organic acid and all the components (surfactant, excipient, binder, and moisture absorbent) other than the carbonate (baking soda) were granulated. In the case of "Organic acid granulated", the carbonate (baking soda) was used as ungranulated starting material particles.

"Carbonate granulated" refers to a state in which the carbonate and all the components (surfactant, excipient, binder, and moisture absorbent) other than the organic acid (citric acid) were granulated. In the case of "Carbonate granulated", the organic acid (citric acid) was used as ungranulated starting material particles.

"Ungranulated" refers to a state in which all the powder components were used without being granulated. *2 Carrageenan was dissolved in purified water and used as an aqueous solution of 1.6% carrageenan. However, the content of carrageenan in Tables 5 and 7 is indicated by a solid content because the purified water evaporates during the production process.

*3 In Tables 5 and 7, "in whole cleansing composition" means "in all particles constituting the powder or granular cleansing composition".

*4 The particle size in Tables 6 and 8 is indicated by a median diameter.

It is evident from Tables 5 and 7 that the powder or granular cleansing compositions of the present Examples are excellent in preservation stability and excellent in texture.

All the cleansing compositions of the present Examples had favorable foamability, foam break, and long-lasting foam.

### Industrial Applicability

The present invention can provide a powder or granular carbonic acid foaming composition and a powder or granular cleansing composition which have high preservation stability, exhibit reduced roughness in texture, and offer favorable feeling in use. The carbonic acid foaming composition can be used in combination with a hair cosmetic such as a shampoo or a conditioning agent so that the hair cosmetic is in a dosage form of foam. The cleansing composition can be used in a face care product such as a face wash, or a body care product such as a hand soap or a body soap.

## Claims

1. A powder or granular carbonic acid foaming composition comprising
a carbonate and an organic acid, wherein
particles having a particle size of 100 µm or larger are contained at 30% by mass or more, and
a content of particles having a particle size of 100 µm or larger and having a hardness of 5 gf or higher is 0.10% by number or less.

2. The powder or granular carbonic acid foaming composition according to claim 1, wherein the particles having a hardness of 5 gf or higher are of 45% by number or less in the particles having a particle size of 100 µm or larger.

3. The powder or granular carbonic acid foaming composition according to claim 1 or 2, further comprising a moisture absorbent.

4. The powder or granular carbonic acid foaming composition according to any one of claims 1 to 3, wherein the organic acid comprises one or more members selected from the group consisting of citric acid, succinic acid, tartaric acid, and ascorbic acid.

5. The powder or granular carbonic acid foaming composition according to any one of claims 1 to 4, wherein the carbonate comprises one or more members selected from the group consisting of sodium carbonate and sodium bicarbonate.

6. The powder or granular carbonic acid foaming composition according to any one of claims 3 to 5, wherein the moisture absorbent is magnesium oxide.

7. The powder or granular carbonic acid foaming composition according to any one of claims 1 to 6, wherein at least a part of the particles having a particle size of 100 µm or larger is granulated particles.

8. The powder or granular carbonic acid foaming composition according to any one of claims 1 to 7, wherein the composition comprises particles (A) comprising the carbonate and particles (B) comprising the organic acid, wherein
the particles (A) are particles containing no organic acid, and the particles (B) are particles containing no carbonate.

9. The powder or granular carbonic acid foaming composition according to claim 8, further comprising an excipient, wherein
the particles (A), the particles (B), or both the particles (A) and the particles (B) comprise the excipient.

10. A method for producing the powder or granular carbonic acid foaming composition according to any one of claims 1 to 9, comprising a step of
granulating at least one of the carbonate and the organic acid.

11. A method for using the powder or granular carbonic acid foaming composition according to any one of claims 1 to 9, comprising bringing the powder or granular carbonic acid foaming composition into contact with a surfactant-containing composition to form the surfactant-containing composition into a dosage form of foam.

12. The method for using a powder or granular carbonic acid foaming composition according to claim 11, wherein the surfactant-containing composition is a hair cosmetic.

13. A hair cosmetic kit comprising the powder or granular carbonic acid foaming composition according to any one of claims 1 to 9 and a surfactant-containing composition.

14. A powder or granular cleansing composition comprising
a carbonate, an organic acid, and a surfactant, wherein
particles having a particle size of 100 µm or larger are contained at 30% by mass or more, and
a content of particles having a particle size of 100 µm or larger and having a hardness of 5 gf or higher is 0.10% by number or less.

15. The powder or granular cleansing composition according to claim 14, wherein the particles having a hardness of 5 gf or higher are of 45% by number or less in the particles having a particle size of 100 µm or larger.

16. The powder or granular cleansing composition according to claim 14 or 15, further comprising a moisture absorbent.

17. The powder or granular cleansing composition according to any one of claims 14 to 16, wherein the organic acid comprises one or more members selected from the group consisting of citric acid, succinic acid, tartaric acid, and ascorbic acid.

18. The powder or granular cleansing composition according to any one of claims 14 to 17, wherein the carbonate comprises one or more members selected from the group consisting of sodium carbonate and sodium bicarbonate.

19. The powder or granular cleansing composition according to any one of claims 16 to 18, wherein the moisture absorbent is magnesium oxide.

20. The powder or granular cleansing composition according to any one of claims 14 to 19, wherein at least a part of the particles having a particle size of 100 µm or larger is granulated particles.

21. The powder or granular cleansing composition according to any one of claims 14 to 20, wherein the composition comprises particles (A) comprising the carbonate and particles (B) comprising the organic acid, wherein
the particles (A) are particles containing no organic acid, and the particles (B) are particles containing no carbonate.

22. The powder or granular cleansing composition according to claim 21, wherein the particles (A), the particles (B), or both the particles (A) and the particles (B) comprise the surfactant.

23. A method for producing the powder or granular cleansing composition according to any one of claims 14 to 22, comprising a step of
granulating at least one of the carbonate and the organic acid.
